# EUROPEAN PATENT APPLICATION

(11) **EP 4 714 947 A1**
(43) Date of publication of application: **25.03.2026**
(21) Application number: 23937064.6
(22) Date of filing: 18.05.2023
(51) Int. Cl.: C07D 401/14, A61K 31/444, A61P 35/00

(54) **CRYSTAL FORM OF PYRIMIDO AZACYCLO COMPOUND, AND PREPARATION METHOD THEREFOR AND USE THEREOF**

(71) Applicant: TYK Medicines, Inc., Huzhou, Zhejiang 313100 (CN)
(72) Inventor: LI, Jun, Huzhou, Zhejiang 313100 (CN); NIU, Chengshan, Huzhou, Zhejiang 313100 (CN); ZHENG, Maolin, Huzhou, Zhejiang 313100 (CN); LIANG, Apeng, Huzhou, Zhejiang 313100 (CN); WU, Yusheng, Huzhou, Zhejiang 313100 (CN)
(74) Representative: dompatent
(86) International application number: PCT/CN2023/095142
(87) International publication number: WO 2024/234384

(57) **Abstract**

The present invention relates to a crystal form of a pyrimido azacyclo compound, and a preparation method therefor and a use thereof. Particularly, disclosed in the present invention are a series of crystal forms of a compound of formula A, wherein the crystal forms have excellent stability. Further disclosed in the present invention are a preparation method for the crystal forms and a use of the crystal forms in the prevention and/or treatment of tumors.

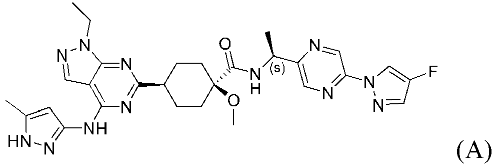

## Description

### FIELD OF THE INVENTION

The present invention relates to the field of pharmaceutical technology, specifically to a crystal form of a pyrimido azacyclo compound, and preparation method therefor and use thereof.

### BACKGROUND OF THE INVENTION

RET (Rearranged during transfection) gene is located on chromosome 10, RET protein encoded thereby is a receptor tyrosine kinase (RTK) which exists on cell membrane. Its mutation types mainly include fusion mutation with KIF5B, TRIM33, CCDC6 and NCOA4 genes, and point mutation at sites such as M918T. Common RET mutations mainly occur in various cancer types, such as thyroid cancer and non-small cell lung cancer. The incidence of RET gene fusion is about 1%-2% in NSCLC patients, and is 10%-20% in papillary thyroid carcinoma (accounting for about 85% of all thyroid cancers). RET fusion is more common in young patients, especially in young non-smoking lung adenocarcinoma patients, and the incidence is as high as 7%-17%. At present, the treatment scheme for RET gene change is mainly to use multi-kinase inhibitors, such as Carbotinib and Vandetanil. Due to the low targeting specificity, serious toxicity related to VEGFR inhibition caused by off-target usually occurs.

Blueprint and Loxo Oncology have disclosed their highly effective and selective oral RET inhibitors BLU-667 and LOXO-292. The results of clinical data from Blueprint show that BLU-667 shows broad anti-tumor activity, and the overall remission rate (ORR) in tumor patients with RET fusion and mutation is 45%, among which the ORR in patients with non-small cell lung cancer and medullary thyroid carcinoma is 50% and 40%, respectively. Recently, FDA granted LOXO-292, a research drug of Loxo Oncology Company, as a breakthrough therapy for treating patients with non-small cell lung cancer (NSCLC) and medullary thyroid cancer (MTC) carrying RET gene mutations.

Therefore, developing novel compounds with RET kinase inhibitory activity and better pharmacodynamic and pharmacokinetic properties has become an important research project to develop new anti-tumor drugs which can be finally used in the treatment of human tumors and other diseases.

Chinese patent application with the publication number CN113698390A discloses a series of compounds as RET kinase inhibitors, which exhibit excellent RET inhibitory activities. A compound with the structural formula shown in formula (A) is disclosed in this patent application. At present, there are no reports of crystal form patents for this compound. The screening and development of crystal forms for the compound of formula (A) to find stable and reliable crystal forms, in order to ensure the quality stability of the compound of formula (A), for better use in pharmaceuticals, drug formulations, and future clinical use, to achieve stable and controllable drug quality, better dissolution, and higher bioavailability, thereby avoiding safety issues such as side effects caused by toxic impurities as a result of drug instability, will be of great significance for later promotion.

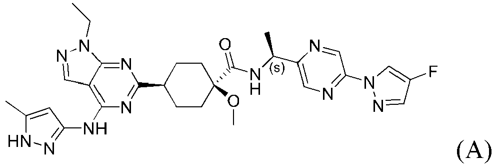

### SUMMARY OF THE INVENTION

The object of the present invention is to provide a crystal form of a pyrimido azacyclo compound, and preparation method therefor and use thereof.

In the first aspect of the present invention, provided is a crystal form of a compound of formula A, wherein the crystal form is selected from the group consisting of free base crystal form III, fumarate crystal form I, and malate crystal form I, 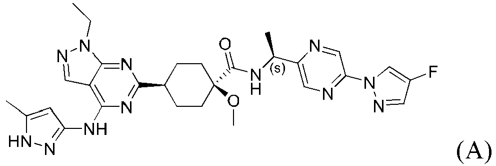 wherein,
the X-ray powder diffraction pattern of the free base crystal form III exhibits characteristic peaks at the following 2θ values: 5.1±0.2°, 5.8±0.2°, 7.3±0.2°, 8.2±0.2°, 9.3±0.2°, 10.4±0.2°, 10.7±0.2°, 13.0±0.2°, 13.3±0.2°, 14.7±0.2°, 16.7±0.2°, 18.4±0.2°, 19.0±0.2° and 23.4±0.2°;
the X-ray powder diffraction pattern of fumarate crystal form I exhibits characteristic peaks at the following 2θ values: 6.0±0.2°, 8.7±0.2°, 10.6±0.2°, 14.6±0.2°, 15.5±0.2°, 17.5±0.2°, 18.0±0.2°, 19.2±0.2° and 27.1±0.2°;
the X-ray powder diffraction pattern of malate crystal form I exhibits characteristic peaks at the following 2θ values: 6.0±0.2°, 9.3±0.2°, 10.7±0.2°, 11.2±0.2°, 11.8±0.2°, 15.3±0.2°, 17.3±0.2°, 17.6±0.2°, 18.3±0.2°, 19.0±0.2°, 23.5±0.2°, 25.7±0.2° and 28.0±0.2°.

In another preferred embodiment,
the X-ray powder diffraction pattern of the free base crystal form III exhibits characteristic peaks at the following 2θ values: 5.1±0.2°, 5.8±0.2°, 7.3±0.2°, 7.8±0.2°, 8.2±0.2°, 9.3±0.2°, 10.4±0.2°, 10.7±0.2°, 11.0±0.2°, 13.0±0.2°, 13.3±0.2°, 14.0±0.2°, 14.7±0.2°, 15.2±0.2°, 16.7±0.2°, 17.5±0.2°, 18.4±0.2°, 19.0±0.2°, 21.1±0.2°, 22.2±0.2°, 23.4±0.2°and 29.5±0.2°;
the X-ray powder diffraction pattern of fumarate crystal form I exhibits characteristic peaks at the following 2θ values: 6.0±0.2°, 8.7±0.2°, 10.6±0.2°, 11.0±0.2°, 11.9±0.2°, 12.1±0.2°, 12.5±0.2°, 14.6±0.2°, 15.5±0.2°, 15.9±0.2°, 17.5±0.2°, 18.0±0.2°, 19.2±0.2°, 26.7±0.2°and 27.1±0.2°;
the X-ray powder diffraction pattern of malate crystal form I exhibits characteristic peaks at the following 2θ values: 6.0±0.2°, 9.3±0.2°, 10.2±0.2°, 10.7±0.2°, 11.2±0.2°, 11.8±0.2°, 13.2±0.2°, 15.3±0.2°, 16.2±0.2°, 17.3±0.2°, 17.6±0.2°, 18.3±0.2°, 19.0±0.2°, 23.5±0.2°, 24.8±0.2°, 25.7±0.2°, 27.6±0.2°and 28.0±0.2°.

In another preferred embodiment,
the X-ray powder diffraction pattern of the free base crystal form III exhibits characteristic peaks at the following 2θ values: 5.1±0.2°, 5.8±0.2°, 7.3±0.2°, 7.8±0.2°, 8.2±0.2°, 9.3±0.2°, 10.4±0.2°, 10.7±0.2°, 11.0±0.2°, 13.0±0.2°, 13.3±0.2°, 14.0±0.2°, 14.7±0.2°, 15.2±0.2°, 16.7±0.2°, 17.5±0.2°, 18.4±0.2°, 19.0±0.2°, 21.1±0.2°, 21.6±0.2°, 22.2±0.2°, 23.4±0.2°, 24.9±0.2°, 25.9±0.2°, 26.3±0.2°, 27.9±0.2°, 28.4±0.2°, 29.5±0.2°, 30.0±0.2°and 35.6±0.2°;
the X-ray powder diffraction pattern of fumarate crystal form I exhibits characteristic peaks at the following 2θ values: 6.0±0.2°, 8.2±0.2°, 8.7±0.2°, 9.9±0.2°, 10.6±0.2°, 11.0±0.2°, 11.9±0.2°, 12.1±0.2°, 12.5±0.2°, 14.6±0.2°, 15.5±0.2°, 15.9±0.2°, 16.5±0.2°, 17.5±0.2°, 18.0±0.2°, 19.2±0.2°, 21.2±0.2°, 22.2±0.2°, 23.9±0.2°, 25.5±0.2°, 26.7±0.2°, 27.1±0.2°and 28.8±0.2°;
the X-ray powder diffraction pattern of malate crystal form I exhibits characteristic peaks at the following 2θ values: 6.0±0.2°, 9.3±0.2°, 10.2±0.2°, 10.7±0.2°, 11.2±0.2°, 11.8±0.2°, 12.1±0.2°, 13.2±0.2°, 15.3±0.2°, 16.2±0.2°, 16.8±0.2°, 17.1±0.2°, 17.3±0.2°, 17.6±0.2°, 18.3±0.2°, 19.0±0.2°, 23.5±0.2°, 24.8±0.2°, 25.2±0.2°, 25.7±0.2°, 27.6±0.2°, 28.0±0.2°and 29.7±0.2°.

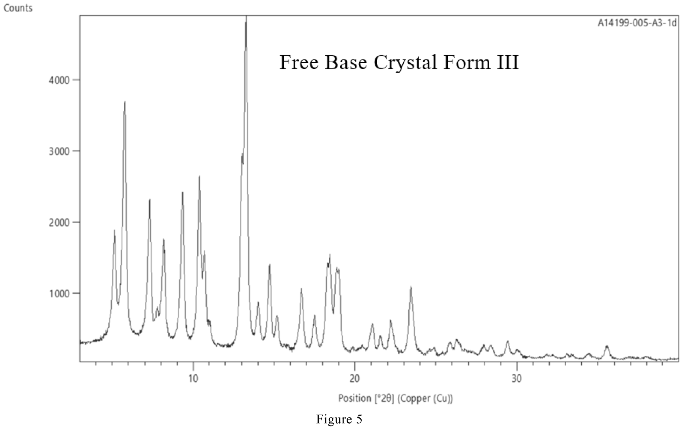

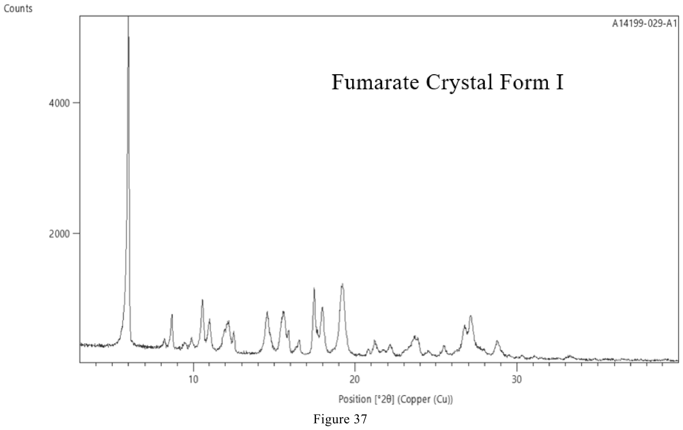

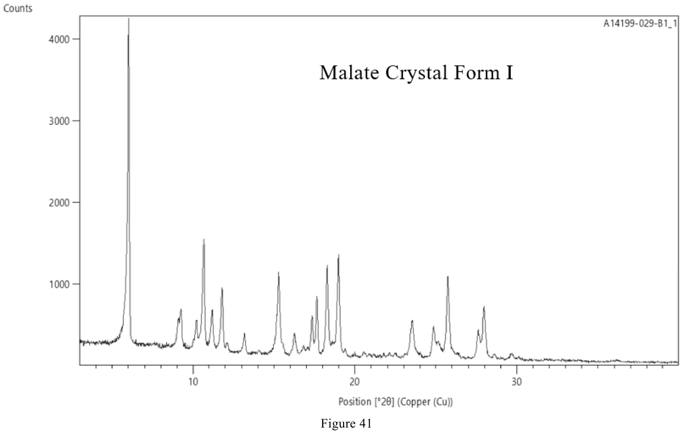

In another preferred embodiment,
the X-ray powder diffraction pattern of the free base crystal form III is substantially shown in Figure 5;
the X-ray powder diffraction pattern of the fumarate crystal form I is substantially shown in Figure 37;
the X-ray powder diffraction pattern of the malate crystal form I is substantially shown in Figure 41.

In another preferred embodiment, the crystal form is selected from the group consisting of fumarate crystal form I and malate crystal form I.

In another preferred embodiment, the crystal form is fumarate crystal form I.

In another preferred embodiment, the fumarate crystal form I is a triclinic system with the P1 space group.

In another preferred embodiment, in the fumarate crystal form I, each asymmetric unit contains two free base molecules and one fumarate molecule, and each unit cell contains one asymmetric unit.

In another preferred embodiment, the unit cell parameters of fumarate crystal form I are a=9.7377(2), b=11.8718(3) Å, c=15.2744(3) Å, α=105.363°, β=91.357°, γ=109.397°, V=1593.89 (7) Å3, Z=1.

In the second aspect of the present invention, provided is a method for preparing free base crystal form III, the method is selected from the group consisting of:
method 1: mixing a compound of formula A with a solvent m, dissolving and clarifying, and crystallizing; the solvent m is selected from one of butanone, acetone, and acetonitrile; and
method 2: mixing a compound of formula A with a solvent n, slurrying at high temperature, and crystallizing; the solvent n is selected from one of ethyl acetate, isopropyl acetate, and methyl tert-butyl ether.

In another preferred embodiment, the compound of formula A is a free base crystal form I.

In another preferred embodiment, the "high temperature" is 50-70 °C.

In the third aspect of the present invention, provided is a method for preparing fumarate crystal form I or malate crystal form I, the method is selected from the group consisting of:
method 1: dissolving a compound of formula A in an organic solvent, stirring at room temperature, then adding fumaric acid or malic acid, continuing stirring, and performing solid-liquid separation to obtain the fumarate crystal form I or malate crystal form I; and
method 2: adding an organic solvent to the fumarate or malate salt of compound A, suspending and stirring, and performing solid-liquid separation to obtain the fumarate crystal form I or malate crystal form I.

In another preferred embodiment, the organic solvent is 2-methyltetrahydrofuran.

In the fourth aspect of the present invention, provided is a use of the crystal form described in the first aspect of the present invention for the manufacture of a medicament, the medicament is used for a use selected from the group consisting of:
1) treating a disease or condition related to RET regulation;
2) treating a tumor; and
3) inhibiting cell proliferation.

In another preferred embodiment, the disease or condition related to RET regulation is selected from the group consisting of thyroid cancer and non-small cell lung cancer.

In another preferred embodiment, the cell is selected from the group consisting of human thyroid cancer cell and human lung cancer cell.

In another preferred embodiment, the tumor is selected from the group consisting of thyroid cancer and non-small cell lung cancer.

It should be understood that within the scope of the present invention, the above-described technical features of the present invention and the technical features described in detail below (e.g., examples) may be combined with each other to constitute a new or preferred technical solution. Limited by space, it will not be repeated here.

### DESCRIPTION OF THE DRAWINGS

Figure 1 is the X-ray powder diffraction pattern of the free base crystal form I of the present invention;
Figure 2 is the DSC-TGA spectrum of the free base crystal form I of the present invention;
Figure 3 is the X-ray powder diffraction pattern of the free base crystal form II of the present invention;
Figure 4 is the DSC-TGA spectrum of the free base crystal form II of the present invention;
Figure 5 is the X-ray powder diffraction pattern of the free base crystal form III of the present invention;
Figure 6 is the DSC-TGA spectrum of the free base crystal form III of the present invention;
Figure 7 is the X-ray powder diffraction pattern of the free base crystal form IV of the present invention;
Figure 8 is the DSC-TGA spectrum of the free base crystal form IV of the present invention;
Figure 9 is the X-ray powder diffraction pattern of the free base crystal form V of the present invention;
Figure 10 is the DSC-TGA spectrum of the free base crystal form V of the present invention;
Figure 11 is the X-ray powder diffraction pattern of the free base crystal form VI of the present invention;
Figure 12 is the DSC-TGA spectrum of the free base crystal form VI of the present invention;
Figure 13 is the X-ray powder diffraction pattern of the free base crystal form VII of the present invention;
Figure 14 is the DSC-TGA spectrum of the free base crystal form VII of the present invention;
Figure 15 is the X-ray powder diffraction pattern of the free base crystal form VIII of the present invention;
Figure 16 is the DSC-TGA spectrum of the free base crystal form VIII of the present invention;
Figure 17 is the X-ray powder diffraction pattern of the free base crystal form IX of the present invention;
Figure 18 is the DSC-TGA spectrum of the free base crystal form IX of the present invention;
Figure 19 is the X-ray powder diffraction pattern of the hydrochloride salt crystal form I of the present invention;
Figure 20 is the DSC-TGA spectrum of the hydrochloride salt crystal form I of the present invention;
Figure 21 is the X-ray powder diffraction pattern of the *p*-toluenesulfonate crystal form I of the present invention;
Figure 22 is the DSC-TGA spectrum of the *p*-toluenesulfonate crystal form I of the present invention;
Figure 23 is the X-ray powder diffraction pattern of the methanesulfonate crystal form I of the present invention;
Figure 24 is the DSC-TGA spectrum of the methanesulfonate crystal form I of the present invention;
Figure 25 is the X-ray powder diffraction pattern of the benzenesulfonate crystal form I of the present invention;
Figure 26 is the DSC-TGA spectrum of the benzenesulfonate crystal form I of the present invention;
Figure 27 is the X-ray powder diffraction pattern of the oxalate crystal form I of the present invention;
Figure 28 is the DSC-TGA spectrum of the oxalate crystal form I of the present invention;
Figure 29 is the X-ray powder diffraction pattern of the oxalate crystal form II of the present invention;
Figure 30 is the DSC-TGA spectrum of the oxalate crystal form II of the present invention;
Figure 31 is the X-ray powder diffraction pattern of the maleate crystal form I of the present invention;
Figure 32 is the DSC-TGA spectrum of the maleate crystal form I of the present invention;
Figure 33 is the X-ray powder diffraction pattern of the phosphate crystal form I of the present invention;
Figure 34 is the DSC-TGA spectrum of the phosphate crystal form I of the present invention;
Figure 35 is the X-ray powder diffraction pattern of the tartrate crystal form I of the present invention;
Figure 36 is the DSC-TGA spectrum of the tartrate crystal form I of the present invention;
Figure 37 is the X-ray powder diffraction pattern of the fumarate crystal form I of the present invention;
Figure 38 is the DSC-TGA spectrum of the fumarate crystal form I of the present invention;
Figure 39 is the X-ray powder diffraction pattern of the citrate crystal form I of the present invention;
Figure 40 is the DSC-TGA spectrum of the citrate crystal form I of the present invention;
Figure 41 is the X-ray powder diffraction pattern of the malate crystal form I of the present invention;
Figure 42 is the DSC-TGA spectrum of the malate crystal form I of the present invention;

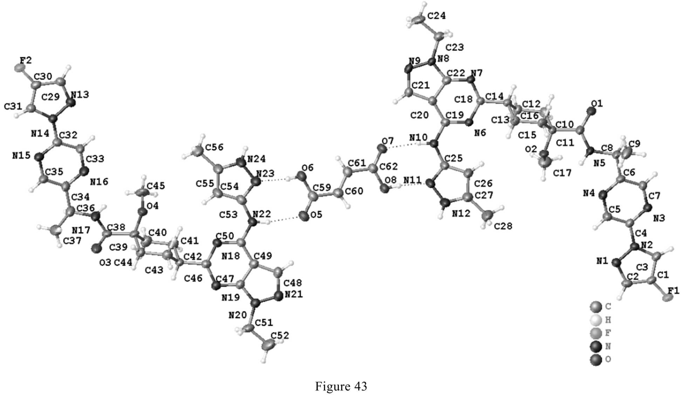

Figure 43 is the single crystal structure diagram of the fumarate crystal form I of the present invention (containing two free base molecules and one fumarate molecule, each unit cell containing one asymmetric unit, and the absolute configuration of carbon (C-8/C-36) is S configuration);
Figure 44 is the X-ray powder diffraction pattern of the amorphous free base of the present invention;
Figures 45 and 46 are PLM diagrams of the free base crystal form I of the present invention.

### DETAILED DESCRIPTION OF THE INVENTION

After long-term and in-depth research, the inventors unexpectedly prepared a series of crystal forms of compound of formula A with excellent stability, especially free base crystal form III, fumarate crystal form I, and malate crystal form I. Based on this, the inventor completed the present invention.

### Crystal form

Crystal form is the solid material state in which drugs exist, and drug crystal form research is the study of the basic state of a drug. Only with a relatively sufficient and comprehensive understanding of a crystal form state of a chemical drug can it be possible to find the drug crystal form solid materials more suitable for treating diseases. The crystal form of a drug can affect its physicochemical properties, directly affecting the clinical efficacy of the drug in treating diseases. The different crystal forms of the same drug may have significant differences in appearance, solubility, melting point, dissolution, and bioavailability, which can affect the stability, bioavailability, and efficacy of the drug. Therefore, studying the stable crystal form of this compound is of great significance.

The active ingredient of the same drug generally exists in two or more crystal forms, which are called drug polymorphs. Different crystal forms show their own different solubility and dissolution rates, which affect the therapeutic effect of the drug in clinical practice by causing changes in their bioavailability *in vivo.* The different crystal forms of a drug may affect their dissolution and absorption in the body, thereby affecting the bioavailability, clinical efficacy, and safety of the drug. Meanwhile, the stability of a drug crystal form is also crucial. In order to improve the bioavailability of a drug, reduce toxicity, and enhance therapeutic efficacy, more attention should be paid to the stability of drug crystal forms. A stable crystal form can ensure the physical and chemical stability of the drug formulation during preparation and storage, maintain good solubility and bioavailability of the drug formulation, and ensure equivalence between each batch of drugs. The same drug often has multiple crystal forms, at present, the most suitable crystal form in clinical practice with better therapeutic effect is called the dominant drug crystal form.

The present invention screened the crystal forms of the compound shown in formula (A) and identified as many different crystal forms of the active pharmaceutical ingredient as possible. The selected crystal forms were identified by powder X-ray diffraction analysis (XRPD), differential scanning calorimetry (DSC), thermogravimetric analysis (TGA), hydrogen spectrum liquid nuclear magnetic resonance (¹H NMR), and high performance liquid chromatography (HPLC). Furthermore, thermodynamic stability and hygroscopicity studies were conducted to determine the dominant drug free base crystal form III, fumarate crystal form I, and malate crystal form I. This provides a reference for crystal form selection in subsequent pharmacokinetic and animal experiments.

Further pharmacokinetic studies have shown that the free base crystal form III, fumarate crystal form I, and malate crystal form I of the compound shown in formula (A) provided by the present invention are easy to absorb and have good stability *in vivo.* They have a good blood drug concentration distribution and high bioavailability *in vivo.* At the same time, due to their good physical stability, they are very beneficial for quality control in subsequent formulation development and longer shelf life of drugs, thereby enabling drugs to achieve better therapeutic effects.

In another preferred embodiment, the compound of formula A further has the crystal form selected from the group consisting of free base crystal form I, free base crystal form II, free base crystal form IV, free base crystal form V, free base crystal form VI, free base crystal form VII, free base crystal form VIII, and free base crystal form IX,
wherein, the X-ray powder diffraction pattern of the free base crystal form I exhibits characteristic peaks at the following 2θ values: 6.8±0.2°, 8.6±0.2°, 9.9±0.2°, 12.9±0.2°, 13.7±0.2° and 18.8±0.2°;
the X-ray powder diffraction pattern of the free base crystal form II exhibits characteristic peaks at the following 2θ values: 5.4±0.2°, 8.1±0.2°, 9.7±0.2°, 10.0±0.2°, 10.5±0.2° and 13.6±0.2°;
the X-ray powder diffraction pattern of the free base crystal form IV exhibits characteristic peaks at the following 2θ values: 5.7±0.2°, 8.3±0.2°, 9.5±0.2°, 11.8±0.2°, 12.2±0.2°, 12.5±0.2°, 12.9±0.2°, 13.4±0.2°, 14.4±0.2°, 15.4±0.2°, 17.3±0.2°, 18.4±0.2°, 18.6±0.2° and 20.2±0.2°;
the X-ray powder diffraction pattern of the free base crystal form V exhibits characteristic peaks at the following 2θ values: 5.4±0.2°, 9.5±0.2°, 9.8±0.2°, 11.9±0.2° and 13.7±0.2°;
the X-ray powder diffraction pattern of the free base crystal form VI exhibits characteristic peaks at the following 2θ values:5.7±0.2°, 7.4±0.2°, 8.4±0.2°, 9.7±0.2°, 10.3±0.2°, 11.5±0.2°, 12.4±0.2°, 14.6±0.2°, 15.7±0.2°, 17.1±0.2°, 17.4±0.2°, 18.7±0.2°, 20.3±0.2° and 23.1±0.2°;
the X-ray powder diffraction pattern of the free base crystal form VII exhibits characteristic peaks at the following 2θ values: 5.1±0.2°, 5.6±0.2°, 7.0±0.2°, 7.4±0.2°, 9.5±0.2°, 9.9±0.2°, 10.3±0.2°, 13.0±0.2°, 13.3±0.2°, 14.4±0.2°, 14.9±0.2°, 16.1±0.2°, 18.1±0.2°, 18.6±0.2°, 18.9±0.2° and 20.6±0.2°;
the X-ray powder diffraction pattern of the free base crystal form VIII exhibits characteristic peaks at the following 2θ values: 5±0.2°, 8.3±0.2°, 8.6±0.2°, 10.1±0.2°, 11.2±0.2°, 12.4±0.2°, 12.6±0.2°, 13.1±0.2°, 13.6±0.2°, 17.7±0.2°, 18±0.2° and 19.2±0.2°; and
the X-ray powder diffraction pattern of the free base crystal form IX exhibits characteristic peaks at the following 2θ values: 7.9±0.2°, 11.8±0.2°, 15.9±0.2°, 16.4±0.2°, 17.3±0.2°, 20.0±0.2°, 20.2±0.2° and 21.2±0.2°.

In another preferred embodiment, the X-ray powder diffraction pattern of the free base crystal form I exhibits characteristic peaks at the following 2θ values: 5.8±0.2°, 6.8±0.2°, 8.6±0.2°, 9.9±0.2°, 12.0±0.2°, 12.9±0.2°, 13.7±0.2°, 14.4±0.2°, 15.2±0.2°, 16.5±0.2°, 17.2±0.2°, 17.6±0.2°, 18.4±0.2°, 18.8±0.2°, 21.9±0.2°, 23.0±0.2° and 23.5±0.2°;
the X-ray powder diffraction pattern of the free base crystal form II exhibits characteristic peaks at the following 2θ values: 5.4±0.2°, 8.1±0.2°, 9.7±0.2°, 10.0±0.2°, 10.5±0.2°, 11.8±0.2°, 12.6±0.2°, 13.6±0.2°, 16.4±0.2°, 19.8±0.2° and 23.0±0.2°;
the X-ray powder diffraction pattern of the free base crystal form IV exhibits characteristic peaks at the following 2θ values: 5.7±0.2°, 6.6±0.2°, 6.7±0.2°, 7.2±0.2°, 8.3±0.2°, 9.5±0.2°, 10.0±0.2°, 11.5±0.2°, 11.8±0.2°, 12.2±0.2°, 12.5±0.2°, 12.9±0.2°, 13.1±0.2°, 13.4±0.2°, 14.4±0.2°, 14.6±0.2°, 15.4±0.2°, 16.1±0.2°, 16.6±0.2°, 17.3±0.2°, 17.5±0.2°, 18.0±0.2°, 18.4±0.2°, 18.6±0.2°, 20.2±0.2°, 22.7±0.2° and 23.0±0.2°;
the X-ray powder diffraction pattern of the free base crystal form V exhibits characteristic peaks at the following 2θ values: 5.4±0.2°, 8.2±0.2°, 9.5±0.2°, 9.8±0.2°, 11.1±0.2°, 11.9±0.2°, 12.8±0.2°, 13.7±0.2°, 16.4±0.2°, 19.2±0.2°, 20.9±0.2° and 23.2±0.2°;
the X-ray powder diffraction pattern of the free base crystal form VI exhibits characteristic peaks at the following 2θ values: 5.7±0.2°, 6.7±0.2°, 7.4±0.2°, 8.4±0.2°, 9.7±0.2°, 10.3±0.2°, 11.5±0.2°, 12.0±0.2°, 12.4±0.2°, 13.0±0.2°, 13.6±0.2°, 14.6±0.2°, 15.7±0.2°, 16.2±0.2°, 17.1±0.2°, 17.4±0.2°, 18.7±0.2°, 20.3±0.2°, 21.4±0.2°, 22.8±0.2°, 23.1±0.2° and 29.9±0.2°;
the X-ray powder diffraction pattern of the free base crystal form VII exhibits characteristic peaks at the following 2θ values: 5.1±0.2°, 5.6±0.2°, 7.0±0.2°, 7.4±0.2°, 7.8±0.2°, 8.7±0.2°, 9.5±0.2°, 9.9±0.2°, 10.3±0.2°, 11.7±0.2°, 12.1±0.2°, 13.0±0.2°, 13.3±0.2°, 13.8±0.2°, 14.4±0.2°, 14.9±0.2°, 16.1±0.2°, 16.3±0.2°, 18.1±0.2°, 18.6±0.2°, 18.9±0.2°, 20.6±0.2° and 23.5±0.2°;
the X-ray powder diffraction pattern of the free base crystal form VIII exhibits characteristic peaks at the following 2θ values: 4.3±0.2°, 5±0.2°, 5.5±0.2°, 8.3±0.2°, 8.6±0.2°, 10.1±0.2°, 10.4±0.2°, 11.2±0.2°, 12.4±0.2°, 12.6±0.2°, 13.1±0.2°, 13.6±0.2°, 14.4±0.2°, 16.6±0.2°, 17.7±0.2°, 18±0.2°, 19.2±0.2°, 20.3±0.2°, 22±0.2°, 23.6±0.2°, 24.9±0.2°, 25.1±0.2°, 26.1±0.2° and 26.4±0.2°; and
the X-ray powder diffraction pattern of the free base crystal form IX exhibits characteristic peaks at the following 2θ values: 7.9±0.2°, 8.4±0.2°, 11.8±0.2°, 13.8±0.2°, 15.9±0.2°, 16.4±0.2°, 17.3±0.2°, 19.0±0.2°, 19.2±0.2°, 20.0±0.2°, 20.2±0.2°, 21.2±0.2° and 28.4±0.2°.

In another preferred embodiment, the X-ray powder diffraction pattern of the free base crystal form I exhibits characteristic peaks at the following 2θ values: 5.8±0.2°, 6.8±0.2°, 8.6±0.2°, 9.9±0.2°, 12.0±0.2°, 12.9±0.2°, 13.7±0.2°, 14.4±0.2°, 15.2±0.2°, 16.5±0.2°, 17.2±0.2°, 17.6±0.2°, 18.1±0.2°, 18.4±0.2°, 18.8±0.2°, 19.5±0.2°, 20.5±0.2°, 21.6±0.2°, 21.9±0.2°, 22.3±0.2°, 23.0±0.2°, 23.5±0.2°, 25.1±0.2°, 25.7±0.2°, 26.1±0.2°, 29.6±0.2° and 29.9±0.2°;
the X-ray powder diffraction pattern of the free base crystal form II exhibits characteristic peaks at the following 2θ values: 5.4±0.2°, 6.6±0.2°, 8.1±0.2°, 9.1±0.2°, 9.7±0.2°, 10.0±0.2°, 10.5±0.2°, 11.4±0.2°, 11.8±0.2°, 12.6±0.2°, 13.6±0.2°, 14.6±0.2°, 16.4±0.2°, 16.8±0.2°, 19.2±0.2°, 19.8±0.2°, 21.1±0.2°, 21.7±0.2°, 22.0±0.2°, 23.0±0.2°, 23.8±0.2°, 25.6±0.2° and 29.4±0.2°;
the X-ray powder diffraction pattern of the free base crystal form IV exhibits characteristic peaks at the following 2θ values: 3.5±0.2°, 4.1±0.2°, 5.3±0.2°, 5.7±0.2°, 6.6±0.2°, 6.7±0.2°, 7.2±0.2°, 8.3±0.2°, 9.5±0.2°, 10.0±0.2°, 10.2±0.2°, 10.7±0.2°, 11.5±0.2°, 11.8±0.2°, 12.2±0.2°, 12.5±0.2°, 12.9±0.2°, 13.1±0.2°, 13.4±0.2°, 14.4±0.2°, 14.6±0.2°, 15.4±0.2°, 15.6±0.2°, 16.1±0.2°, 16.6±0.2°, 16.7±0.2°, 17.3±0.2°, 17.5±0.2°, 18.0±0.2°, 18.4±0.2°, 18.6±0.2°, 18.9±0.2°, 19.7±0.2°, 19.9±0.2°, 20.2±0.2°, 20.7±0.2°, 21.0±0.2°, 21.1±0.2°, 21.9±0.2°, 22.2±0.2°, 22.7±0.2°, 23.0±0.2°, 23.6±0.2°, 24.5±0.2°, 25.2±0.2°, 25.4±0.2°, 25.9±0.2°, 26.1±0.2° and 27.5±0.2°;
the X-ray powder diffraction pattern of the free base crystal form V exhibits characteristic peaks at the following 2θ values: 5.4±0.2°, 8.2±0.2°, 9.5±0.2°, 9.8±0.2°, 11.1±0.2°, 11.9±0.2°, 12.8±0.2°, 13.7±0.2°, 14.9±0.2°, 16.4±0.2°, 17.1±0.2°, 18.7±0.2°, 19.2±0.2°, 20.3±0.2°, 20.9±0.2°, 22.0±0.2°, 22.4±0.2°, 23.2±0.2°, 25.3±0.2°, 25.8±0.2°, 26.4±0.2°, 26.9±0.2°, 28.1±0.2°, 29.0±0.2° and 30.1±0.2°;
the X-ray powder diffraction pattern of the free base crystal form VI exhibits characteristic peaks at the following 2θ values: 4.2±0.2°, 5.7±0.2°, 6.7±0.2°, 7.4±0.2°, 8.4±0.2°, 9.7±0.2°, 10.3±0.2°, 11.5±0.2°, 12.0±0.2°, 12.4±0.2°, 13.0±0.2°, 13.6±0.2°, 14.6±0.2°, 15.7±0.2°, 16.2±0.2°, 17.1±0.2°, 17.4±0.2°, 18.7±0.2°, 20.3±0.2°, 21.0±0.2°, 21.4±0.2°, 22.8±0.2°, 23.1±0.2°, 25.8±0.2°, 26.3±0.2°, 27.2±0.2°, 28.8±0.2°, 29.2±0.2° and 29.9±0.2°;
the X-ray powder diffraction pattern of the free base crystal form VII exhibits characteristic peaks at the following 2θ values: 5.1±0.2°, 5.6±0.2°, 7.0±0.2°, 7.4±0.2°, 7.8±0.2°, 8.7±0.2°, 9.5±0.2°, 9.9±0.2°, 10.3±0.2°, 11.4±0.2°, 11.7±0.2°, 12.1±0.2°, 13.0±0.2°, 13.3±0.2°, 13.8±0.2°, 14.4±0.2°, 14.9±0.2°, 15.5±0.2°, 16.1±0.2°, 16.3±0.2°, 16.9±0.2°, 18.1±0.2°, 18.6±0.2°, 18.9±0.2°, 19.7±0.2°, 20.6±0.2°, 21.2±0.2°, 21.8±0.2°, 22.5±0.2°, 23.1±0.2°, 23.5±0.2°, 24.9±0.2° and 25.4±0.2°;
the X-ray powder diffraction pattern of the free base crystal form VIII exhibits characteristic peaks at the following 2θ values: 4.3±0.2°, 5±0.2°, 5.5±0.2°, 6.7±0.2°, 8.3±0.2°, 8.6±0.2°, 9.7±0.2°, 10.1±0.2°, 10.4±0.2°, 11.2±0.2°, 12.4±0.2°, 12.6±0.2°, 13.1±0.2°, 13.6±0.2°, 14.4±0.2°, 15±0.2°, 15.6±0.2°, 16.6±0.2°, 17.7±0.2°, 18±0.2°, 18.6±0.2°, 19.2±0.2°, 20.3±0.2°, 21±0.2°, 21.3±0.2°, 22±0.2°, 22.6±0.2°, 23.1±0.2°, 23.6±0.2°, 24.9±0.2°, 25.1±0.2°, 25.7±0.2°, 26.1±0.2°, 26.4±0.2°, 27±0.2°, 27.5±0.2°, 28.9±0.2°, 30.3±0.2° and 31.8±0.2°;
the X-ray powder diffraction pattern of the free base crystal form IX exhibits characteristic peaks at the following 2θ values: 5.5±0.2°, 5.8±0.2°, 6.8±0.2°, 7.9±0.2°, 8.4±0.2°, 8.6±0.2°, 9.8±0.2°, 11.8±0.2°, 13.3±0.2°, 13.8±0.2°, 14.0±0.2°, 15.9±0.2°, 16.4±0.2°, 17.3±0.2°, 17.6±0.2°, 19.0±0.2°, 19.2±0.2°, 20.0±0.2°, 20.2±0.2°, 20.6±0.2°, 21.2±0.2°, 24.2±0.2°, 27.8±0.2°, 28.4±0.2° and 29.4±0.2°.

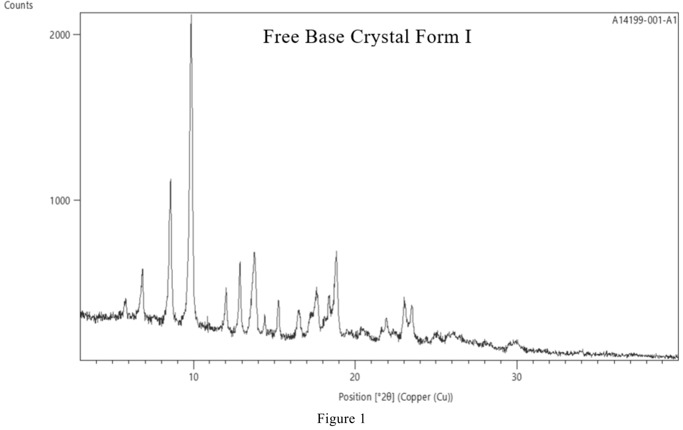

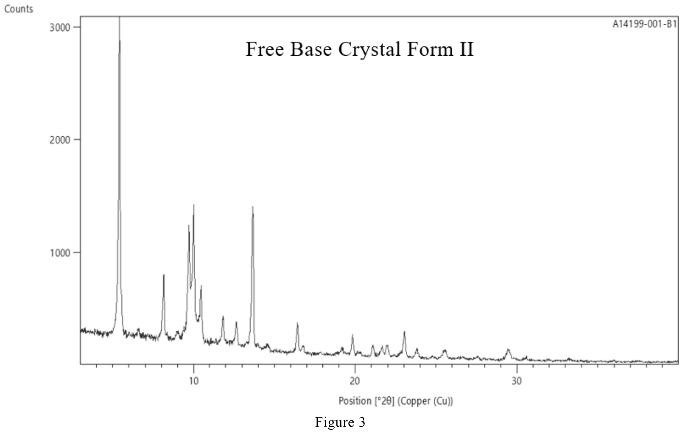

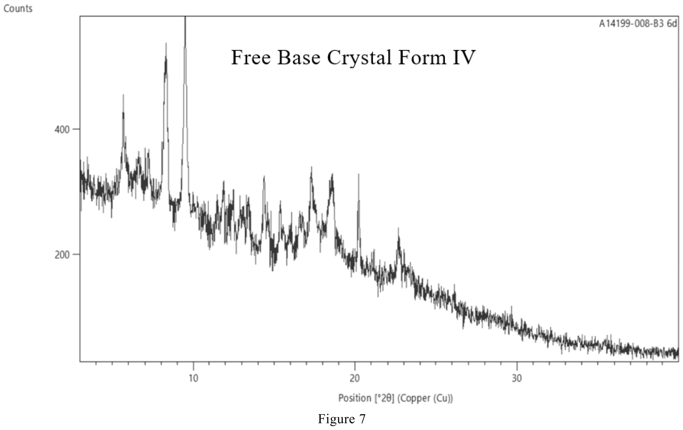

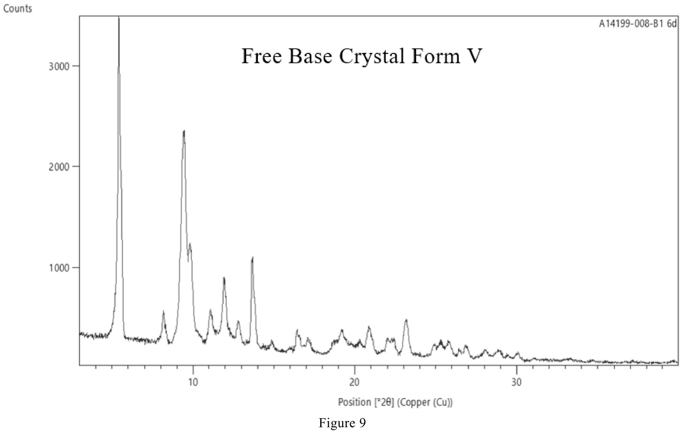

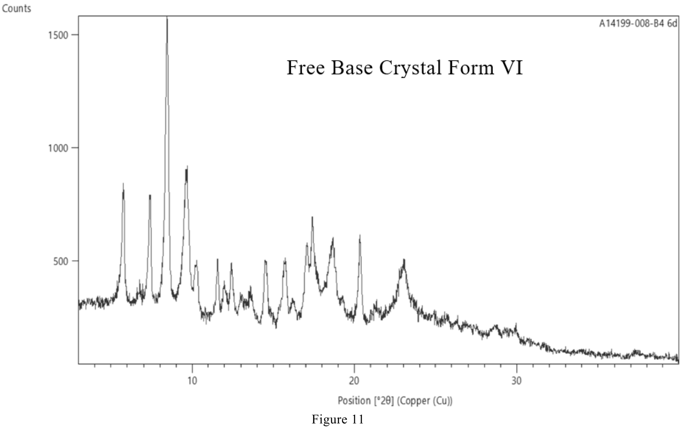

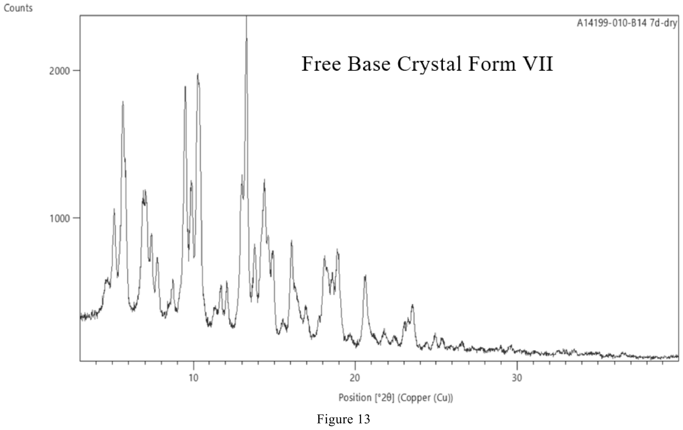

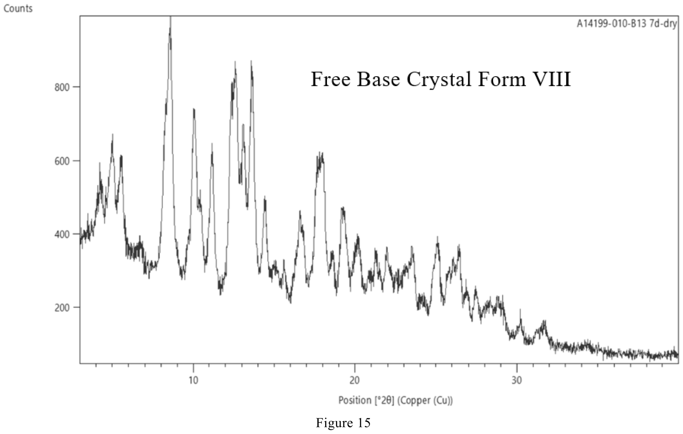

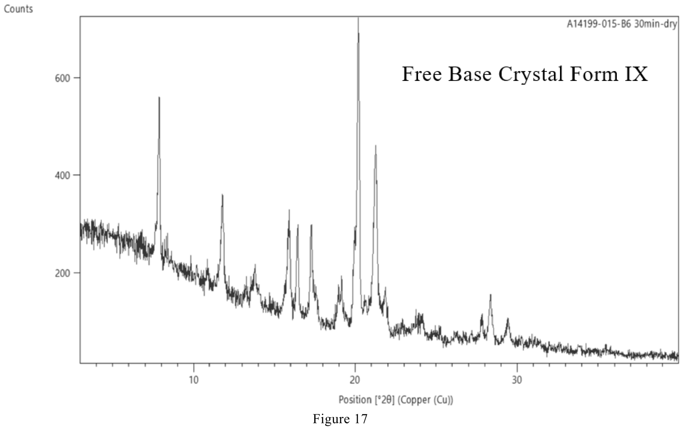

In another preferred embodiment, the X-ray powder diffraction pattern of the free base crystal form I is substantially shown in Figure 1;
The X-ray powder diffraction pattern of the free base crystal form II is substantially shown in Figure 3;
The X-ray powder diffraction pattern of the free base crystal form IV is substantially shown in Figure 7;
The X-ray powder diffraction pattern of the free base crystal form V is substantially shown in Figure 9;
The X-ray powder diffraction pattern of the free base crystal form VI is substantially shown in Figure 11;
The X-ray powder diffraction pattern of the free base crystal form VII is substantially shown in Figure 13;
The X-ray powder diffraction pattern of the free base crystal form VIII is substantially shown in Figure 15; and
The X-ray powder diffraction pattern of the free base crystal form IX is substantially shown in Figure 17.

Compared with the prior art, the present invention has the following main advantages:
1) The crystal forms have excellent stability and high solubility;
2) The free base crystal form III, fumarate crystal form I, and malate crystal form I of the present invention have better pharmacokinetic properties and higher animal exposure levels compared to the free base crystal form I.

The present invention will be further explained below in combination with specific examples. It should be understood that these examples are only used to illustrate the present invention and not to limit the scope of the present invention. In the following examples, the test methods without specific conditions are usually in accordance with conventional conditions or the conditions recommended by the manufacturer. Unless otherwise specified, percentages and parts are calculated by weight.

Unless otherwise defined, all professional and scientific terms used herein have the same meanings as those familiar to those skilled in the field. In addition, any method and material similar or equivalent to those described herein can be applied to the method of the present invention. The preferred embodiment methods and materials described herein are for demonstration purposes only.

The abbreviations in this invention are shown in the table below

### Chemical reagent abbreviation:

- 2-Me-THF: 2-methyl tetrahydrofuran
- ACN: acetonitrile
- DCM: dichloromethane
- DMSO: dimethyl sulfoxide
- EA: ethyl acetate
- EtOH: ethanol
- Hept: *n*-heptane
- IPA: isopropanol
- IPAc: isopropyl acetate
- MEK: butanone
- MeOH: methanol
- MTBE: methyl *tert*-butyl ether
- n-BuOH: *n*-butanol
- THF: tetrahydrofuran
- Tol: toluene
- HCL: hydrochloric acid
- H₂SO₄: sulfuric acid
- TS: *p*-toluenesulfonic acid
- MS: methanesulfonic Acid
- BS: benzenesulfonic acid
- OX: oxalic acid
- MA: maleic acid
- H₃PO₄: phosphoric acid
- TA: L-tartaric acid
- FU: fumaric acid
- CI: citric acid monohydrate

### Other abbreviations (arranged in alphabetical order):

- DSC: differential scanning calorimetry
- DVS: dynamic water vapor adsorption
- HPLC: High Performance Liquid Chromatography
- NMR: Nuclear Magnetic Resonance Mass Spectrometry
- PLM: polarizing microscope
- RT: room temperature (20-25 °C)
- TGA: thermogravimetric analysis
- XRPD: X-ray powder diffraction
- Fasting simulated intestinal fluid: FaSSIF
- Fed-state simulated intestinal fluid: FeSSIF
- Simulated Gastric Fluid: Simulated Gastric Fluid

The following provides a detailed explanation of the technical solution of the present invention.

### I. Synthesis of the compound shown in formula (A)

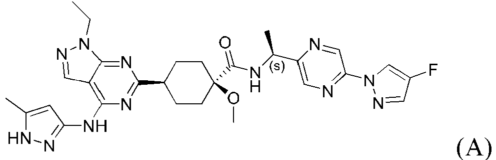

The preparation method is the same as that in CN113698390A, and the synthesis route is as follows:

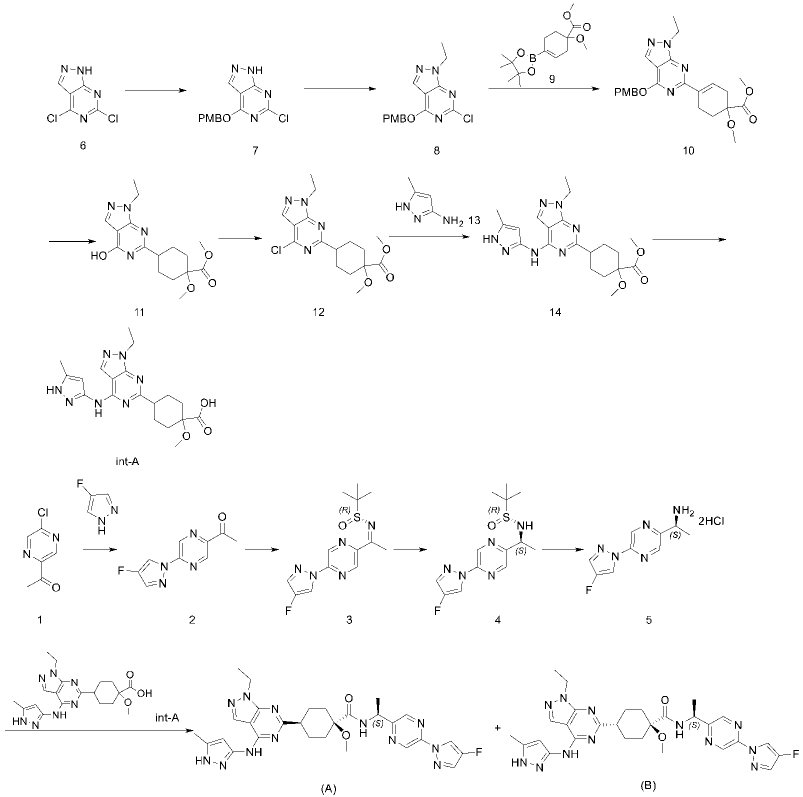

The specific steps were as follows:

### Synthesis of Compound 7

p-Methoxybenzyl alcohol (16.0 g, 116.4 mmol) and potassium *tert*-butoxide (24.9 g, 222.2 mmol) were dissolved in 400 mL of anhydrous dioxane, and compound 6 (20.0 g, 105.8 mmol) dissolved in dioxane (300 mL) was added dropwise to the reaction flask in an ice bath. The solution was gradually turned into colloidal form under magnetic stirring for 2 h. After the reaction was completed, the reaction was quenched with water, the aqueous phase was extracted with ethyl acetate, and the separated organic phase was washed with saturated brine, dried over anhydrous sodium sulfate, rotary evaporated to dryness under reduced pressure to obtain the crude product, which was then slurried with petroleum ether for two hours, filtered, and dried under vacuum to obtain 28.8 g of compound 7, MS [M+H] 291.

¹H NMR (400 MHz, Chloroform-*d*) δ 8.07 (s, 1H), 7.47 (d, *J* = 8.7 Hz, 2H), 6.95 (d, J= 8.7 Hz, 2H), 5.56 (s, 2H), 3.84 (s, 3H).

### Synthesis of Compound 8

Compound 7 (2.0 g, 6.80 mmol) was dissolved in 60 mL of anhydrous THF, ethanol (1.1 mL, 17.2 mmol) and triphenylphosphine (2.9 g, 11.0 mmol) were added sequentially, the reaction solution was protected by nitrogen, diisopropyl azodicarboxylate (2.8 g, 13.6 mmol) was added in an ice bath, the mixture was warmed up to room temperature naturally and stirred for 3 h. The reaction was quenched by adding saturated brine, then extracted with ethyl acetate, the organic phase was separated and washed with saturated brine, dried over anhydrous sodium sulfate, and concentrated under reduced pressure to obtain the crude product, which was purified by column chromatography to obtain 1.78 g of compound 8, MS [M+H] 319.

¹H NMR (400 MHz, Chloroform-*d*) δ 7.99 (s, 1H), 7.45 (d, *J* = 8.8 Hz, 2H), 6.94 (d, *J* = 8.7 Hz, 2H), 5.54 (s, 2H), 4.45 (q, *J* = 7.2 Hz, 2H), 3.83 (s, 3H), 1.50 (t, *J* = 7.3 Hz, 3H).

### Synthesis of Compound 10

Compound 8 (1.78 g, 5.60 mmol) and compound 9 (1.82 g, 6.16 mmol) were dissolved in a mixed solvent of 60mL of 1,4-dioxane and 12 mL of water, and palladium acetate (126 mg, 0.56 mmol), 1,1'-bis(diphenylphosphine) ferrocene (621 mg, 1.12 mmol) and potassium carbonate (1.16 g, 8.40 mmol) were added sequentially. The reaction solution was stirred at 90°C under nitrogen protection overnight. After the reaction was completed, the reaction solution was cooled to room temperature, concentrated under reduced pressure, and extracted with ethyl acetate. The combined organic phase was washed with saturated brine, dried over anhydrous sodium sulfate, concentrated under reduced pressure and purified by column chromatography to obtain 1.52 g of compound C10. MS [M+H] 453.

¹H NMR (400 MHz, Chloroform-*d*) δ 7.93 (s, 1H), 7.42 (s, 2H), 7.35-7.29 (m, 1H), 6.91 (d, *J* = 8.7 Hz, 2H), 5.55 (s, 2H), 4.46 (q, *J* = 7.3 Hz, 2H), 3.81 (d, *J* = 2.9 Hz, 6H), 3.33 (s, 3H), 2.97 - 2.49 (m, 4H), 2.41 - 2.20 (m, 1H), 2.20 - 2.08 (m, 1H), 1.50 (t, J= 7.2 Hz, 3H).

### Synthesis of Compound 11

Compound 10 (1.52 g, 3.36 mmol) was dissolved in a mixed solvent of 15 mL ethyl acetate and 15 mL methanol, then wet palladium on carbon (152 mg) was added, the mixture was stirred overnight at room temperature under hydrogen atmosphere. After the reaction was completed, the reaction solution was filtered through diatomite, washed with methanol, and the filtrate was concentrated under reduced pressure to obtain 1.33 g of Compound 11, MS [M+H] 335.

¹H NMR (400 MHz, Chloroform-*d*) δ 10.64 (s, 1H), 8.00 (s, 1H), 4.33 (q, *J* = 7.1 Hz, 2H), 3.73 (s, 3H), 3.24 (s, 3H), 2.71 - 2.60 (m, 1H), 2.14 (d, *J* = 12.4 Hz, 2H), 1.97 - 1.72 (m, 6H), 1.44 (t, *J* = 7.2 Hz, 3H).

### Synthesis of Compound 12

Compound 11 (1.33 g, crude) was dissolved in phosphorus oxychloride (30 mL) and stirred at 100 °C for 3 h under nitrogen protection. After the reaction was completed, the solvent was evaporated under reduced pressure, and crushed ice was added. The mixture was adjusted to neutral with saturated sodium bicarbonate solution, extracted with ethyl acetate, and the combined organic phases were washed with saturated brine, dried over anhydrous sodium sulfate, concentrated under reduced pressure, and purified by column chromatography to obtain 936 mg of compound 12, MS [M+H] 353.

¹H NMR (400 MHz, Chloroform-*d*) δ 8.00 (s, 1H), 4.46 (q, *J* = 7.3 Hz, 2H), 3.73 (s, 3H), 3.24 (s, 3H), 3.09 - 2.84 (m, 1H), 2.13 (d, *J* = 11.8 Hz, 2H), 2.00 - 1.72 (m, 6H), 1.47 (t, *J* = 7.3 Hz, 3H).

### Synthesis of Compound 14

Compound 12 (936 mg, 2.66 mmol) and compound 13 (284 mg, 2.92 mmol) were dissolved in 15 mL of dimethyl sulfoxide, then diisopropylethylamine (686 mg, 5.32 mmol) was added, the mixture was stirred overnight at 100 °C under nitrogen protection. After the reaction was completed, the reaction mixture was cooled to room temperature, and diluted with ethyl acetate. The organic phase was washed with saturated brine, dried over anhydrous sodium sulfate, concentrated under reduced pressure and purified by column chromatography to obtain 728 mg of compound 14. MS [M+H] 414.

¹H NMR (400 MHz, Chloroform-*d*) δ 7.78 (s, 1H), 6.54 (s, 1H), 4.44 (q, *J* = 6.3, 5.3 Hz, 2H), 3.80 (s, 3H), 3.33 (s, 3H), 2.93 - 2.82 (m, 1H), 2.36 (s, *J* = 6.5 Hz, 3H), 2.23 - 1.62 (m, 8H), 1.49 (t, *J* = 7.2 Hz, 3H).

### Synthesis of Compound int-A

Compound 14 (728 mg, 1.76 mmol) was dissolved in a mixed solvent of 16 mL of methanol and 2 mL of water, lithium hydroxide monohydrate (222 mg, 5.29 mmol) was added, the mixture was stirred at 60 °C overnight. After the reaction was completed, the reaction solution was cooled to room temperature, evaporated under reduced pressure to remove methanol, slurried with DCM, filtered by suction filtration to obtain 797 mg of crude product int-A, which was used directly in the next step without purification, MS [M+H] 400.

### Synthesis of Compound 2

Compound 1 (1.57 g, 10 mmol), 4-fluoro-1H pyrazole (860 mg, 10 mmol), N,N-dimethylformamide (10 mL), and potassium carbonate (2.76 g, 20 mmol) were added to a 50 mL single necked flask, and the reaction mixture was reacted at 100 °C for 8 hours. LC-MS showed that the reaction was completed, and then the reaction mixture was poured into water (200 mL) with stirring to precipitate a solid. The mixture was filtered and the filter cake was washed with water, then the filter cake was dried to obtain 1.08 g of compound 2. [M+1]:207.03.

### Synthesis of Compound 3

Compound 2 (870 mg, 4.2 mmol), *R*-*tert*-butyl sulfonamide (560 mg, 4.63 mmol), tetraethyl titanate (1.92 g, 8.4 mmol), and dry tetrahydrofuran (10 mL) were added in a 100 mL single necked flask, and the reaction mixture was reacted overnight at 75 °C. The mixture was cooled, diluted with water (30 mL), filtered, the filtrate was extracted three times with ethyl acetate (20 mL), the organic phase was dried over anhydrous sodium sulfate, evaporated to dryness under reduced pressure and purified by column chromatography to obtain 500 mg of product.

### Synthesis of Compound 4

Compound 3 (100 mg, 0.32 mmol) and dry tetrahydrofuran (2 mL) were sequentially added to a 50 mL single necked flask, cooled to -78 °C, and tris (2-butyl) lithium borohydride (0.7 mL, 0.7 mmol, 1.0M) was added dropwise. The mixture was reacted at -78 °C for 1h. The reaction was quenched with the addition of methanol (1 mL) at -60 °C, and the mixture was diluted with water (5 mL), and extracted three times with ethyl acetate (5 mL). The organic phase was dried, evaporated to dryness under reduced pressure, and subjected to column chromatography to obtain 93 mg of the product.

¹H NMR (400 MHz, CDCl₃) δ 9.23 (d, J = 1.4 Hz, 1H), 8.36 (d, J = 1.5 Hz, 1H), 8.33 (d, J = 4.6 Hz, 1H), 7.66 (d, J = 4.3 Hz, 1H), 4.77 (p, J = 6.6 Hz, 1H), 3.74 (d, J = 5.9 Hz, 1H), 1.67 (d, J = 6.8 Hz, 3H), 1.22 (s, 9H)_{∘}

### Synthesis of Compound 5

Compound 4 (100 mg, 0.32 mmol), dichloromethane (3 mL), methanol (0.5 mL), and hydrogen chloride/dioxane (4M, 1.5 mL) were sequentially added to a 50 mL single necked flask and the reaction mixture was reacted at room temperature for 3 hours. LC-MS detected the reaction was completed, then the mixture was diluted with dichloromethane and filtered. The filter cake was washed with ethyl acetate, and dried to obtain 67 mg of product. [M+1]: 208.06.

### Synthesis of Formula (A)

Compound int-A (270 mg, 0.68 mmol) and compound 5 (165 mg, 0.68 mmol) were dissolved in 9 mL of N,N-dimethylformamide, diisopropylethylamine (699 mg, 5.41 mmol) was added, the mixture was stirred at room temperature for 15 minutes, then HATU (386 mg, 1.02 mmol) was added and continued stirring at room temperature for 1 hour. The reaction solution was diluted with ethyl acetate, the organic phase was washed with saturated brine, dried over anhydrous sodium sulfate, concentrated, and purified by high-performance liquid chromatography to obtain formula (A), Ms [M+H] 589, and formula (B), MS [M+H] 589

Nuclear magnetic of formula (A): ¹H NMR (400 MHz, Chloroform-d) δ 9.23 (d, J = 1.4 Hz, 1H), 8.37 (d, J = 1.4 Hz, 1H), 8.34 (d, J = 4.6 Hz, 1H), 7.78 (s, 1H), 7.66 (d, J = 4.3 Hz, 2H), 5.32 (p, J = 6.8 Hz, 1H), 4.44 (q, J = 7.2 Hz, 2H), 3.32 (s, 3H), 2.93 - 2.77 (m, 1H), 2.38 (s, 3H), 2.13 - 1.90 (m, 8H), 1.56 (d, J = 6.9 Hz, 3H), 1.48 (t, J = 7.2 Hz, 3H).

The obtained solid of formula (A) was characterized by XRPD, and the result was shown in Figure 1. The obtained solid is the free base crystal form I.

The free base crystal form I was used as a raw material for the preparation of crystal form and the preparation of salt crystal form experiments described below.

### II. Preparation and evaluation of the crystal form of the compound of formula (A)

At room temperature, the solubility of free base crystal form I in 17 solvents was evaluated by visual inspection. 5 mg of free base crystal form I was weighed and added to an 8 mL bottle, solvent was gradually added dropwise until the drug was completely dissolved or reached 1600V, and the solubility (mg/mL) was roughly calculated. The results were shown in Table 1. Free base crystal form I exhibited high solubility (> 40 mg/mL) in DMSO and THF, but poor solubility in the remaining solvents.

**Table 1 Rough solubility results of free base crystal form I**

| # | Solvent | Solubility (mg/mL) |
|---|---|---|
| 1 | MeOH | ~ 1.2 |
| 2 | EtOH | <0.6 |
| 3 | IPA | <0.6 |
| 4 | n-BuOH | <0.6 |
| 5 | MEK | 6.3~7.1 |
| 6 | Acetone | 7.1~8.3 |
| 7 | IPAc | 1.5~1.6 |
| 8 | EA | 2.2~2.3 |
| 9 | ACN | -0.8 |
| 10 | THF | 41.7~50.0 |
| 11 | 2-Me-THF | 11.1~12.5 |
| 12 | DCM | 4.6~5.0 |
| 13 | MTBE | <0.6 |
| 14 | Tol | <0.6 |
| 15 | Hept | <0.6 |
| 16 | DMSO | >166.7 |
| 17 | Water | <0.6 |

| | | |
|---|---|---|
| If not dissolved, marked as "<"; if dissolved, marked as ">". | | |

### 1. Crystal form screening method

The solid crystal form screening of compound (A) involved crystallizing or precipitating from selected single solvents and their mixed solvents to obtain metastable and stable crystal forms. The methods used included volatilization crystallization, slurry crystallization, cooling crystallization, and anti-solvent precipitation.

### 1.1 Volatilization Crystallization

Volatilization crystallization was conducted in the selected solvent based on solubility data. At room temperature or 50 °C, 5 mg of free base crystal form I was dissolved in a solvent (0.5 mL) and stirred at RT or 50 °C for 20 minutes to form a clear solution. The solution was divided into two parts, one part was dried with nitrogen for rapid evaporation, and the other part was covered with a perforated film and slowly evaporated at room temperature.

### 1.2 Slurry Crystallization

30 mg of free base crystal form I was weighed and added into a sample bottle, solvent was added slowly to form a suspension. The mixture was stirred at RT or 50 °C for 7 days, or at 70 °C for 2 days, filtered and solids were collected, and the filter cake was characterized by XRPD.

### 1.3 Cooling Crystallization

15 mg of free base crystal form I was weighed and added into a sample bottle, solvent (0.5 mL) was added slowly, and the mixture was stirred at 50 °C or 40 °C (DCM) for 10 minutes. The solution was close to clarification, and the mother liquor was obtained by microporous filtration. The solution was evenly divided into two parts, one part of the mother liquor was placed in a refrigerator (~4 °C) for rapid cooling, and the other part was placed in a heated metal module and naturally cooled to room temperature. If the solid precipitated, it was characterized immediately.

### 1.4 Anti-solvent Precipitation

At room temperature, 15-25 mg of free base crystal form I was dissolved in THF (0.5 mL) or DMSO (0.25 mL) and filtered to obtain a clear solution. Anti-solvent was gradually added with 0.1-1 mL each time until the solid precipitated or the volume of solvent added reached 10V. If the solid precipitated, it was characterized immediately.

### 2. Crystal form analysis method

### 2.1 Polarizing Microscope (PLM) Analysis

PLM analysis was performed using ECLIPSE LV100POL (Nikon, JPN) polarizing microscope. A small amount of sample was spread flat on a glass slide, cedar oil was dropped to disperse the sample, and then a cover glass was covered. Subsequently, the sample was placed under a microscope and observed with a 4-20 x objective lens.

### 2.2 X-ray Powder Diffraction (XRPD)

X-ray diffractometer was used to detect solid samples. The sample was spread flat on a zero-background monocrystalline silicon sample disk, gently pressed and flatten, and analyzed according to the parameters in Table 2.

**Table 2 XRPD Test Parameters**

| | |
|---|---|
| Instrument | PANalytical, Empyrean |
| Light source | Cu Kα (λ = 1.5418 Å) |
| Detector | PIXcel^{1D} |
| Scanning angle | 3-40° (2θ) |
| Scanning step size | 0.013° (2θ) |
| Scanning speed | 20.4 s/step |
| Light tube voltage/current | 45 kV/40 mA |
| Divergence slit | 1/8° |
| Rotate | open |
| Sample Disk | Zero Background Sample Disk |

### 2.3 Thermogravimetric Analysis (TGA)

The TGA data of the sample was collected using a TA instrument. 1-5 mg of the sample was placed in an open aluminum sample tray that has been tared, the sample was heated and tested according to the instrument parameters in Table 3, and the data was analyzed and processed using TRIOS software.

**Table 3. TGA Test Parameters**

| | |
|---|---|
| Instrument | TA, Discovery TGA 55 |
| Sample tray | Perforated aluminum tray |
| Temperature range | RT - 300 °C |
| Heating rate | 10 °C/min |
| Blow gas | N₂ |
| Flow rate | Balance Room: 40 mL/min |
| | Sample Room: 60 mL/min |

### 2.4 Differential Scanning Calorimetry (DSC) Analysis

The DSC data of the sample was collected using a TA instrument. 1-3 mg of the sample was placed in a DSC sample tray that has been perforated, heating measurement was performed according to the parameters in Table 4, and the data was analyzed and processed using TRIOS software.

**Table 4. DSC Test Parameters**

| | |
|---|---|
| Instrument | TA, Discovery DSC 250 |
| Sample tray | Perforated aluminum tray |
| Temperature range | 25-300 °C |
| Heating rate | 10 °C/min |
| Blow gas | N₂ |
| Flow rate | 50 mL/min |

### 3. Crystal form analysis results

### 3.1 Free Base Crystal Form I

The crystal forms obtained by slurrying with MeOH, EtOH, and IPA systems at room temperature and 50 °C high-temperature, as well as anti-solvent precipitation in IPA/THF systems, are all free base crystal form I.

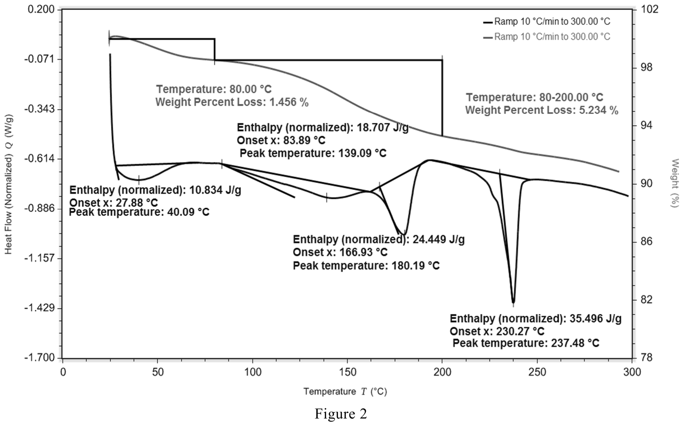

The XRPD result of free base crystal form I is shown in Figure 1, and the DSC and TGA results are shown in Figure 2. 1H-NMR shows that the sample contained 5.4% ethanol, 0.8% n-heptane, and unknown solvent residue. Meanwhile, TGA shows a 1.5% weight loss before 80 °C and a 5.2% weight loss between 80-200 °C, attributed to solvent removal; DSC shows multiple endothermic peaks before 250 °C, attributed to solvent removal and sample melting.

From Figure 1, it can be seen that the X-ray powder diffraction peak data of free base crystal form I is shown in Table 5.

**Table 5**

| 2θ (°) | Relative intensity (%) |
|---|---|
| 5.8 | 6.2 |
| 6.8 | 16.3 |
| 8.6 | 46.1 |
| 9.9 | 100.0 |
| 12.0 | 13.9 |
| 12.9 | 24.2 |
| 13.7 | 26.0 |
| 14.4 | 6.0 |
| 15.2 | 11.7 |
| 16.5 | 8.8 |
| 17.2 | 7.0 |
| 17.6 | 14.0 |
| 18.1 | 4.1 |
| 18.4 | 12.3 |
| 18.8 | 26.5 |
| 19.5 | 2.2 |
| 20.5 | 3.0 |
| 21.6 | 3.6 |
| 21.9 | 6.8 |
| 22.3 | 3.0 |
| 23.0 | 13.8 |
| 23.5 | 11.5 |
| 25.1 | 3.4 |
| 25.7 | 2.6 |
| 26.1 | 3.4 |
| 29.6 | 2.9 |
| 29.9 | 3.5 |

### 3.2 Free Base Crystal Form II

Free base crystal form I was dissolved in a system of trifluoroacetic acid, acetonitrile, and water, the solution was adjusted to alkaline with sodium bicarbonate, the organic solvent was concentrated under reduced pressure, the suspension was filtered, and the solid was washed with water to obtain free base crystal form II.

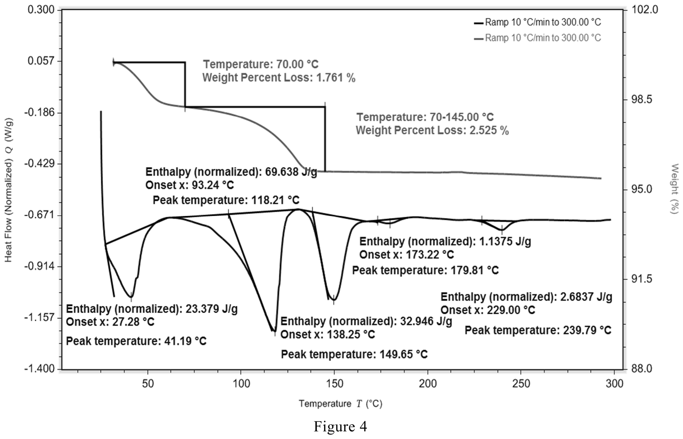

1H-NMR shows that the sample contained 0.2% residual n-hexane, while TGA shows 1.8% weight loss before 70 °C and 2.5% weight loss between 70-145 °C, attributed to the removal of water or solvent. The DSC curve shows multiple endothermic peaks before 200 °C, attributed to solvent removal and sample melting. Therefore, it was speculated that the free base crystal form II is a hydrate crystal form. The X-ray powder diffraction patterns (XRPD) obtained by Cu-Kα ray measurement and DSC-TGA spectra are shown in Figures 3 and 4.

From Figure 3, it can be seen that the X-ray powder diffraction peak data of free base crystal form II is shown in Table 6.

**Table 6**

| 2θ (°) | Relative intensity (%) |
|---|---|
| 5.4 | 100.0 |
| 6.6 | 2.6 |
| 8.1 | 20.2 |
| 9.1 | 2.8 |
| 9.7 | 36.3 |
| 10.0 | 42.4 |
| 10.5 | 16.8 |
| 11.4 | 2.1 |
| 11.8 | 8.8 |
| 12.6 | 7.7 |
| 13.6 | 44.5 |
| 14.6 | 2.1 |
| 16.4 | 9.7 |
| 16.8 | 2.7 |
| 19.2 | 2.2 |
| 19.8 | 6.7 |
| 21.1 | 3.7 |
| 21.7 | 3.5 |
| 22.0 | 3.6 |
| 23.0 | 8.2 |
| 23.8 | 3.1 |
| 25.6 | 2.9 |
| 29.4 | 3.5 |

### 3.3 Free Base Crystal Form III

The crystal forms obtained by slurrying with *n*-butanol, acetone, and acetonitrile at room temperature or 50/70 °C high-temperature, as well as slurrying with ethyl acetate, isopropyl acetate, and methyl *tert*-butyl ether at high-temperature of 50/70 °C, are all free base crystal form III.

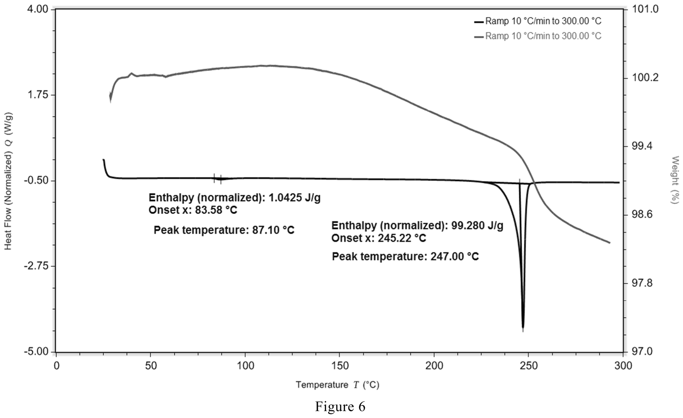

Free base crystal form III is an agglomerated microcrystal with high crystallinity. The XRPD result of free base crystal form III is shown in Figure 5, and the DSC and TGA results are shown in Figure 6. TGA shows that there is no significant weight loss before 150 °C. 1H-NMR shows that the sample contains 0.03% acetone residue. There is a small endothermic peak at 84 °C on the DSC curve (the crystal form remains unchanged when heated to 100 °C). There is another endothermic peak at 245 °C, which is the melting point of the compound. Therefore, the free base crystal form III is an anhydrous crystal form.

From Figure 5, it can be seen that the X-ray powder diffraction peak data of free base crystal form III is shown in Table 7.

**Table 7**

| 2θ (°) | Relative intensity (%) |
|---|---|
| 5.1 | 32.3 |
| 5.8 | 70.4 |
| 7.3 | 38.0 |
| 7.8 | 8.7 |
| 8.2 | 27.8 |
| 9.3 | 44.5 |
| 10.4 | 50.6 |
| 10.7 | 27.9 |
| 11.0 | 6.2 |
| 13.0 | 56.8 |
| 13.3 | 100.0 |
| 14.0 | 10.8 |
| 14.7 | 23.5 |
| 15.2 | 8.7 |
| 16.7 | 18.8 |
| 17.5 | 9.8 |
| 18.4 | 28.5 |
| 19.0 | 24.1 |
| 21.1 | 8.2 |
| 21.6 | 4.2 |
| 22.2 | 9.4 |
| 23.4 | 20.7 |
| 24.9 | 2.5 |
| 25.9 | 4.0 |
| 26.3 | 4.5 |
| 27.9 | 3.4 |
| 28.4 | 3.2 |
| 29.5 | 5.0 |
| 30.0 | 2.3 |
| 35.6 | 4.2 |

### 3.4 Free Base Crystal Form IV

Crystal form IV was prepared by rapid/slow volatilization crystallization in 2-Me-THF or rapid evaporation in THF.

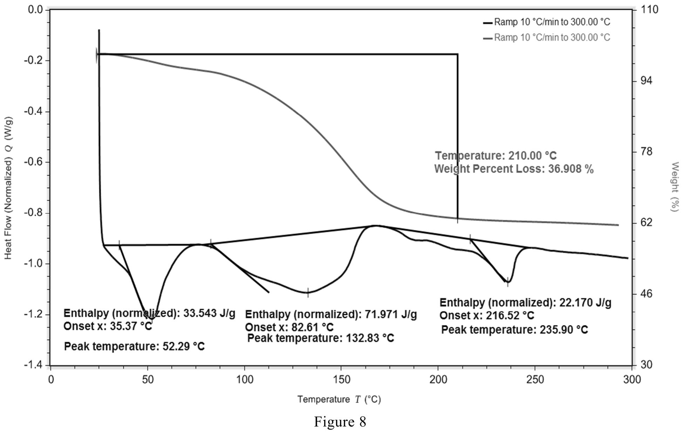

Free base crystal form IV is an agglomerated small crystal with low crystallinity. The XRPD result of free base crystal form IV is shown in Figure 7, and the DSC and TGA results are shown in Figure 8. TGA shows a weight loss of 36.9% before 210 °C, attributed to solvent removal. DSC shows multiple endothermic peaks before 250 °C. After heating to 170 °C, it become amorphous. Therefore, crystal form IV is a hydrate or solvate.

From Figure 7, it can be seen that the X-ray powder diffraction peak data of free base crystal form IV is shown in Table 8.

**Table 8**

| 2θ (°) | Relative intensity (%) |
|---|---|
| 3.5 | 13.8 |
| 4.1 | 10.9 |
| 5.3 | 12.5 |
| 5.7 | 47.5 |
| 6.6 | 16.3 |
| 6.7 | 15.9 |
| 7.2 | 19.4 |
| 8.3 | 82.8 |
| 9.5 | 100.0 |
| 10.0 | 16.3 |
| 10.2 | 14.1 |
| 10.7 | 11.3 |
| 11.5 | 20.3 |
| 11.8 | 26.6 |
| 12.2 | 22.5 |
| 12.5 | 23.8 |
| 12.9 | 22.5 |
| 13.1 | 19.7 |
| 13.4 | 23.4 |
| 14.4 | 37.2 |
| 14.6 | 19.4 |
| 15.4 | 30.3 |
| 15.6 | 11.3 |
| 16.1 | 20.0 |
| 16.6 | 15.6 |
| 16.7 | 12.2 |
| 17.3 | 37.8 |
| 17.5 | 16.6 |
| 18.0 | 15.9 |
| 18.4 | 30.9 |
| 18.6 | 38.1 |
| 18.9 | 7.5 |
| 19.7 | 6.6 |
| 19.9 | 5.3 |
| 20.2 | 48.8 |
| 20.7 | 10.3 |
| 21.0 | 7.2 |
| 21.1 | 5.3 |
| 21.9 | 6.3 |
| 22.2 | 8.4 |
| 22.7 | 22.2 |
| 23.0 | 19.7 |
| 23.6 | 6.9 |
| 24.5 | 10.9 |
| 25.2 | 8.8 |
| 25.4 | 5.9 |
| 25.9 | 8.1 |
| 26.1 | 12.2 |
| 27.5 | 6.9 |

### 3.5 Free Base Crystal Form V

Free base crystal form V was obtained by slowly volatilizing in MEK or DCM systems, cooling crystallization or slurrying with DCM.

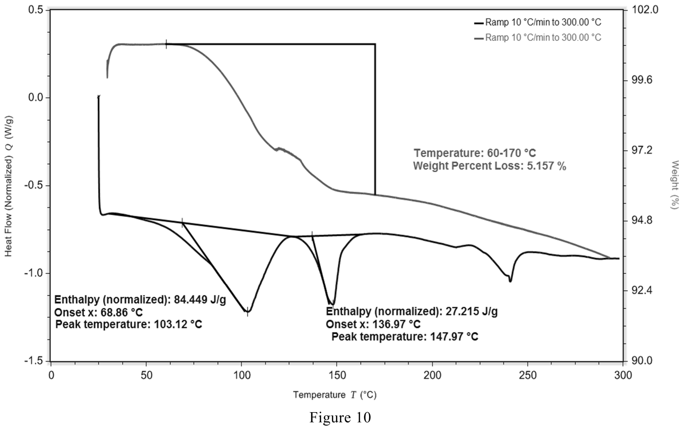

The samples obtained from the MEK system was characterized. The free base crystal form V is a needle-shaped crystal with moderate crystallinity. The XRPD result of the free base crystal form V is shown in Figure 9, and the DSC and TGA results are shown in Figure 10. The TGA curve shows a 5.2% weight loss between 60-170 °C, attributed to solvent removal. DSC shows multiple endothermic peaks before 250 °C, and after heating to 175 °C, the free base crystal form V is transformed into amorphous form. Therefore, the free base crystal form V is a hydrate or solvate.

From Figure 9, it can be seen that the X-ray powder diffraction peak data of free base crystal form V is shown in Table 9.

**Table 9**

| 2θ (°) | Relative intensity (%) |
|---|---|
| 5.4 | 100.0 |
| 8.2 | 9.7 |
| 9.5 | 64.6 |
| 9.8 | 29.5 |
| 11.1 | 8.4 |
| 11.9 | 17.8 |
| 12.8 | 5.9 |
| 13.7 | 27.9 |
| 14.9 | 2.8 |
| 16.4 | 6.9 |
| 17.1 | 4.2 |
| 18.7 | 3.1 |
| 19.2 | 6.6 |
| 20.3 | 2.2 |
| 20.9 | 7.1 |
| 22.0 | 4.8 |
| 22.4 | 4.8 |
| 23.2 | 11.1 |
| 25.3 | 4.8 |
| 25.8 | 3.7 |
| 26.4 | 2.1 |
| 26.9 | 3.8 |
| 28.1 | 2.3 |
| 29.0 | 2.2 |
| 30.1 | 2.4 |

### 3.6 Free Base Crystal Form VI

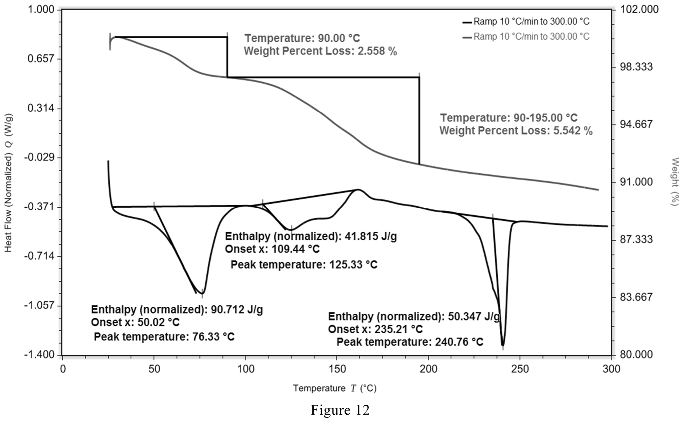

The free base crystal form VI was prepared by the slow volatilizing in THF and characterized. The free base crystal form VI is an agglomerated small crystal with moderate crystallinity. The DSC and TGA results are shown in Figure 12. TGA shows a 2.6% weight loss before 90 °C and a 5.5% weight loss between 90-195 °C, attributed to solvent removal. After heating to 195 °C, the free base crystal form VI is transformed into amorphous form. DSC shows multiple endothermic peaks before 250 °C, indicating that the free base crystal form VI may be a solvate or hydrate.

From Figure 11, it can be seen that the X-ray powder diffraction peak data of free base crystal form VI is shown in Table 10.

**Table 10**

| 2θ (°) | Relative intensity (%) |
|---|---|
| 4.2 | 3.1 |
| 5.7 | 42.6 |
| 6.7 | 8.0 |
| 7.4 | 38.6 |
| 8.4 | 100.0 |
| 9.7 | 49.3 |
| 10.3 | 15.0 |
| 11.5 | 17.7 |
| 12.0 | 12.0 |
| 12.4 | 16.8 |
| 13.0 | 6.9 |
| 13.6 | 11.5 |
| 14.6 | 22.4 |
| 15.7 | 22.6 |
| 16.2 | 7.4 |
| 17.1 | 23.4 |
| 17.4 | 29.7 |
| 18.7 | 22.8 |
| 20.3 | 30.7 |
| 21.0 | 4.2 |
| 21.4 | 6.7 |
| 22.8 | 13.3 |
| 23.1 | 20.1 |
| 25.8 | 4.6 |
| 26.3 | 4.5 |
| 27.2 | 3.5 |
| 28.8 | 4.1 |
| 29.2 | 3.7 |
| 29.9 | 5.4 |

### 3.7 Free Base Crystal Form VII

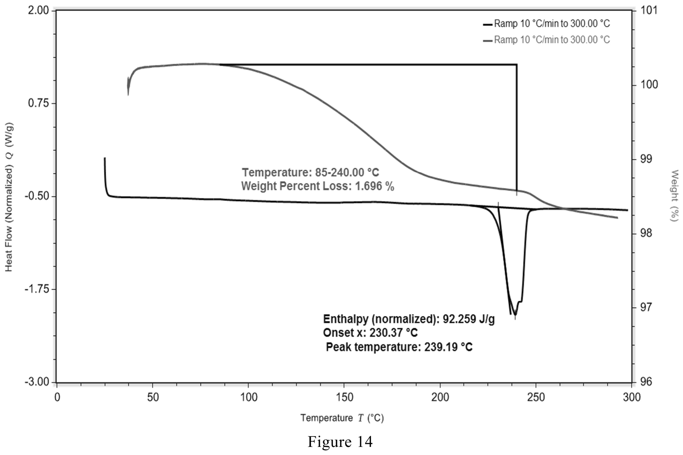

Free base crystal form VII was obtained by slurrying with a 2-Me-THF system at room temperature or high temperature of 50 °C for 7 days. Free base crystal form VII is an agglomerated small particle crystal with moderate crystallinity. 1H-NMR shows 0.97% residual 2-Me-THF in the sample. The DSC and TGA results are shown in Figure 14. TGA shows a weight loss of 1.7% between 85-240 °C, attributed to solvent removal. DSC shows two overlapping endothermic peaks at 230 °C, which are attributed to the melting point. The free base crystal form VII did not change after heating to 210 °C. Therefore, the free base crystal form VII is an anhydrous crystal form.

From Figure 13, it can be seen that the X-ray powder diffraction peak data of free base crystal form VII is shown in Table 11.

**Table 11**

| 2θ (°) | Relative intensity (%) |
|---|---|
| 5.1 | 34.2 |
| 5.6 | 65.5 |
| 7.0 | 40.6 |
| 7.4 | 26.3 |
| 7.8 | 13.3 |
| 8.7 | 11.1 |
| 9.5 | 75.4 |
| 9.9 | 47.0 |
| 10.3 | 84.4 |
| 11.4 | 6.8 |
| 11.7 | 14.4 |
| 12.1 | 14.7 |
| 13.0 | 48.4 |
| 13.3 | 100.0 |
| 13.8 | 18.0 |
| 14.4 | 52.2 |
| 14.9 | 28.2 |
| 15.5 | 4.7 |
| 16.1 | 29.2 |
| 16.3 | 15.0 |
| 16.9 | 7.2 |
| 18.1 | 28.3 |
| 18.6 | 21.9 |
| 18.9 | 31.7 |
| 19.7 | 3.1 |
| 20.6 | 24.7 |
| 21.2 | 3.3 |
| 21.8 | 5.1 |
| 22.5 | 3.2 |
| 23.1 | 8.6 |
| 23.5 | 15.2 |
| 24.9 | 6.0 |
| 25.4 | 3.5 |

### 3.8 Free Base Crystal Form VIII

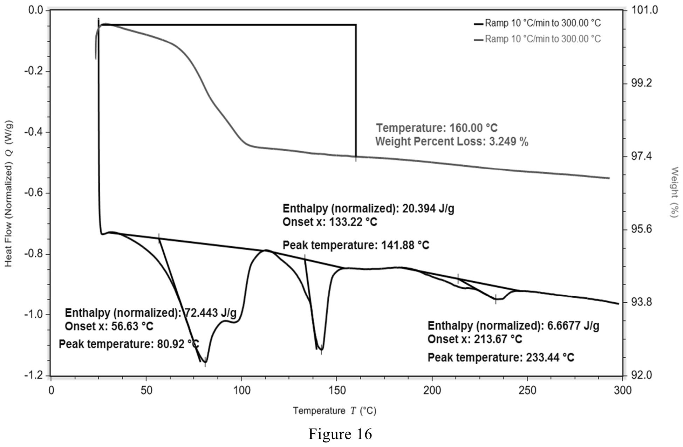

Free base crystal form VIII was obtained by slurrying in 50 °C of water for 7 days. The XRPD spectra of the wet and dry samples are not completely consistent, and there is a certain shift in the peak of the free base crystal form VIII in different states. The DSC and TGA results are shown in Figure 16. After the sample was dried at 40 °C, TGA result shows that there is still over 3.2% weight loss before 160 °C. DSC shows multiple endothermic peaks before 250 °C. 1H-NMR did not detect any significant residual solvent. The compound was heated to 180 °C and transformed into an amorphous form, therefore, the free base form VIII may be a hydrate.

From Figure 15, it can be seen that the X-ray powder diffraction peak data of dried free base crystal form VIII is shown in Table 12

**Table 12**

| 2θ (°) | Relative intensity (%) |
|---|---|
| 4.3 | 18.0 |
| 5.0 | 37.7 |
| 5.5 | 31.6 |
| 6.7 | 8.2 |
| 8.3 | 67.2 |
| 8.6 | 100.0 |
| 9.7 | 13.3 |
| 10.1 | 62.6 |
| 10.4 | 30.4 |
| 11.2 | 52.1 |
| 12.4 | 65.5 |
| 12.6 | 71.6 |
| 13.1 | 54.5 |
| 13.6 | 66.2 |
| 14.4 | 32.9 |
| 15.0 | 7.2 |
| 15.6 | 11.4 |
| 16.6 | 31.5 |
| 17.7 | 48.5 |
| 18.0 | 51.7 |
| 18.6 | 12.4 |
| 19.2 | 33.3 |
| 20.3 | 16.5 |
| 21.0 | 5.9 |
| 21.3 | 12.3 |
| 22.0 | 15.0 |
| 22.6 | 4.8 |
| 23.1 | 13.9 |
| 23.6 | 17.3 |
| 24.9 | 16.7 |
| 25.1 | 26.7 |
| 25.7 | 13.3 |
| 26.1 | 18.3 |
| 26.4 | 26.1 |
| 27.0 | 5.9 |
| 27.5 | 12.4 |
| 28.9 | 8.9 |
| 30.3 | 8.9 |
| 31.8 | 10.1 |

### 3.9 Free Base Crystal Form IX

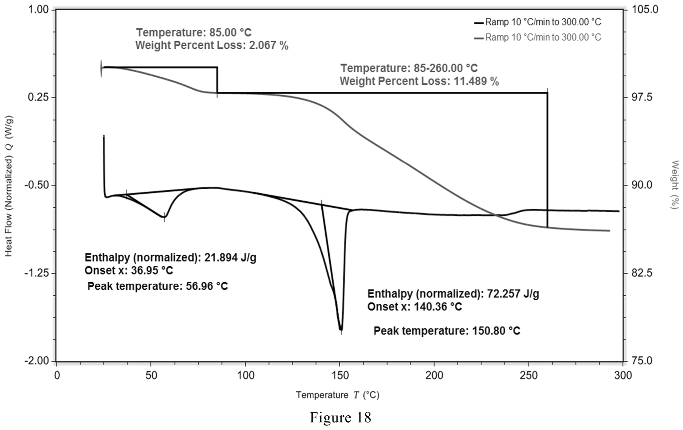

Free base crystal form IX was obtained from the anti-solvent precipitation of DMSO/H₂O. Free base crystal form IX is a small particle crystal with moderate crystallinity. 1H-NMR shows 11.54% DMSO residue. The DSC and TGA results are shown in Figure 18. TGA shows a weight loss of 2.1% before 85 °C and 11.5% between 85-260 °C, attributed to solvent removal. DSC shows a broad endothermic peak at 37 °C and another endothermic peak at 140 °C. Therefore, the free base crystal form IX may be a DMSO solvate.

From Figure 17, it can be seen that the X-ray powder diffraction peak data of free base crystal form IX is shown in Table 13.

**Table 13**

| 2θ (°) | Relative intensity (%) |
|---|---|
| 5.5 | 5.5 |
| 5.8 | 6.1 |
| 6.8 | 7.0 |
| 7.9 | 56.6 |
| 8.4 | 10.4 |
| 8.6 | 5.8 |
| 9.8 | 8.7 |
| 11.8 | 29.4 |
| 13.3 | 9.1 |
| 13.8 | 13.8 |
| 14.0 | 7.6 |
| 15.9 | 34.1 |
| 16.4 | 28.3 |
| 17.3 | 30.9 |
| 17.6 | 8.7 |
| 19.0 | 14.2 |
| 19.2 | 18.1 |
| 20.0 | 30.6 |
| 20.2 | 100.0 |
| 20.6 | 5.8 |
| 21.2 | 57.6 |
| 24.2 | 7.3 |
| 27.8 | 9.1 |
| 28.4 | 15.2 |
| 29.4 | 8.6 |

### 3.10 Free base amorphous

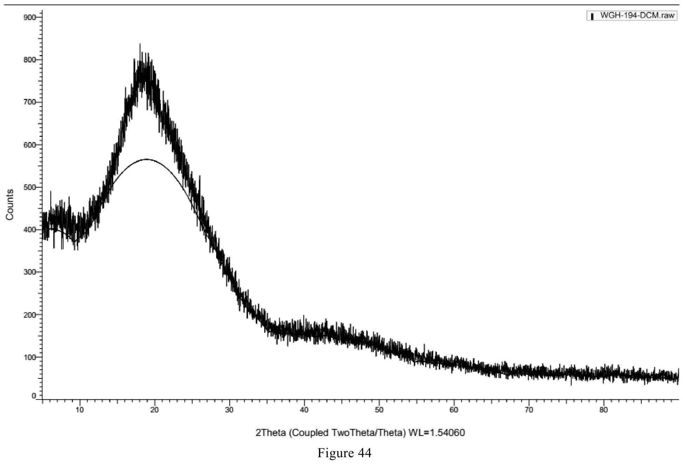

The free base crystal form I sample was weighed, dissolved in 20 mL of dichloromethane at room temperature, filtered, and the filtrate was rotary evaporated to remove the solvent rapidly. The solid sample obtained was subjected to corresponding characterization tests, and its XRD spectrum is shown in Figure 44.

### 4. Conclusion of the Study on Free Base Crystal Forms:

**Table 14**

| Crystal form | DSC Onset/Pk T (°C), ΔH (J/g) | TGA Wt. loss%/ T (°C) | Remarks |
|---|---|---|---|
| Crystal form I solvate | 4 endothermic peaks | 1.5/RT-80 | 5.4% ethanol, 0.8% *n*-heptane and unknown solvent |
| | | 5.2/80-200 | |
| Crystal form II hydrate | 5 endothermic peaks | 1.8/RT-70 | 0.2% *n*-heptane |
| | | 2.5/70-145 | |
| Crystal form III anhydrous crystal form | 245/247, 99 | ~0/RT-150 | No obvious solvent residue |
| Crystal form IV hydrate / solvate | Multiple endothermic peaks | 36.9/RT-210 | Become amorphous upon heating to 170 °C |
| Crystal form V hydrate / solvate | Multiple endothermic peaks | 5.2/60-170 | Become amorphous upon heating to 175 °C |
| Crystal form VI hydrate / solvate | Multiple endothermic peaks | 2.6/RT-90 | Become amorphous upon heating to 195 °C |
| | | 5.5/90-195 | |
| Crystal form VII anhydrous crystal form | 230/239, 92 | 1.7/85-240 | Maintain unchanged in crystal form after heating to 195 °C, possibly due to wrapped in residual solvent |
| | Two adjacent peaks | | |
| Crystal form VIII hydrate | Multiple endothermic peaks | 3.2/RT-160 | Become amorphous upon heating to 180 °C |
| Crystal form IX solvate | 37/57, 22 | 2.1/RT-85 | 11.54% DMSO |
| | 140/151, 72 | 11.5/85-260 | |

The compound with the structure shown in formula (A) exhibits relatively complex polymorphic phenomena in polymorphic screening, with a total of 9 crystal forms obtained, namely crystal forms I-IX. Two of them are anhydrous (crystal form III and crystal form VII), and crystal form III is relatively stable with a higher melting point and enthalpy, while the others are solvates or hydrates. The data summary is shown in Table 14.

In the present invention, the 2θ angle value of the X-ray powder diffraction peak has an error range of ± 0.2°. It should be understood that the 2θ value of X-ray powder diffraction patterns may vary slightly between machines and samples, and the numerical range may differ by ± 0.2 units. Therefore, the quoted values cannot be interpreted as absolute values.

Free base crystal form III is an anhydrous crystalline form with a high melting point (245 °C), and free base crystal form III samples obtained by different solvents or methods are all small particle crystals.

The free base crystal form III provided by the present invention has stable physicochemical properties and is highly suitable for further applications in drug development such as formulation.

The crystal forms I to IX provided by the present invention can be used for the preparation of RET inhibitor drugs, especially crystal form III.

### III. Preparation and characterization of acid salts of the compound shown in formula (A)

The 12 acids used in the salt crystal form screening of the present invention are shown in Table 15.

**Table 15 Acid List**

| Label | Acid |
|---|---|
| 1 | Hydrochloric acid (HCl) |
| 2 | Sulfuric acid (H₂SO₄) |
| 3 | *p*-Toluenesulfonic acid (TS) |
| 4 | Methanesulfonic acid (MS) |
| 5 | Benzenesulfonic acid (BS) |
| 6 | Oxalic acid (OX) |
| 7 | Phosphoric acid (H₃PO₄) |
| 8 | Maleic acid (MA) |
| 9 | L-tartaric acid (TA) |
| 10 | Fumaric acid (FU) |
| 11 | Citric acid (CI) |
| 12 | L-malic acid |

30 mg of free base (i.e. the free base crystal form I prepared above) was added to different solvents at room temperature, and then acid solution or solid acid (1.1 eq. or 0.6 eq.) was added to form a crystalline salt. If there was no solid precipitation, different crystallization methods were used to prepare crystalline salts, such as cooling crystallization, volatilization crystallization, and anti-solvent precipitation. The resulting mixture was filtered and solid was collected, vacuum dried and further analyzed.

### 1. Preparation and characterization of acid salt crystal forms

### 1.1 Hydrochloride Salt Crystal Form I

At room temperature, about 30 mg of free base crystal form I was added to acetone (1.0 ml). Hydrochloric acid solution (1.1 eq., 0.56 mol/L, concentrated hydrochloric acid dissolved in 0.1 mL corresponding solvent) was added to this suspension. After HCl solution was added, the suspension turned yellow and gelatinous. After 10 minutes, the solid precipitated, the mixture was stirred for another 2 days, and subjected to solid-liquid separation to obtain hydrochloride crystal form I.

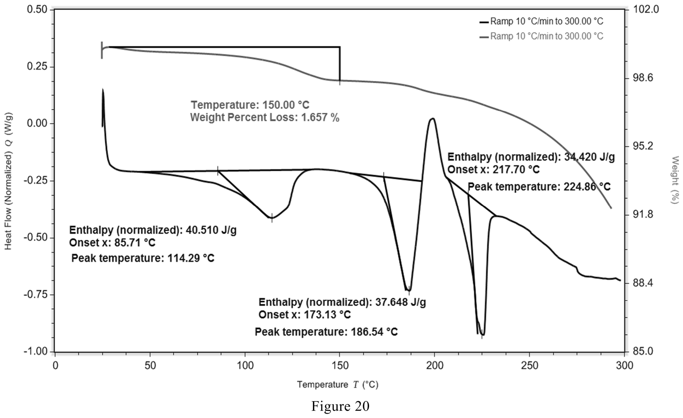

The hydrochloride crystal form I is an agglomerated small crystal with moderate crystallinity. 1H-NMR shows no significant residual organic solvents in the sample. The DSC and TGA results are shown in Figure 20. TGA shows a weight loss of 1.7% before 150 °C, indicating the removal of water. There is a broad endothermic peak in the initial stage of DSC heating, and two other endothermic peaks were detected at 173 °C and 218 °C. Therefore, the hydrochloride crystal form I may be a hydrate.

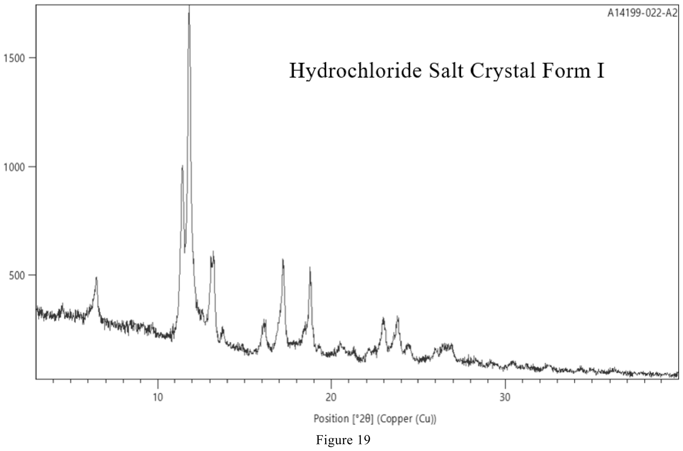

The powder X-ray diffraction pattern is shown in Figure 19, and the data is shown in Table 16:

**Table 16**

| 2θ (°) | Relative intensity (%) | 2θ (°) | Relative intensity (%) |
|---|---|---|---|
| 4.5 | 4.2 | 18.5 | 8.5 |
| 6.5 | 14.3 | 18.8 | 25.4 |
| 8.5 | 3.4 | 20.5 | 4.9 |
| 11.4 | 51.4 | 22.1 | 2.9 |
| 11.8 | 100.0 | 23.0 | 10.9 |
| 12.6 | 3.4 | 23.8 | 11.0 |
| 13.2 | 24.6 | 24.5 | 3.9 |
| 13.7 | 5.2 | 26.4 | 5.1 |
| 16.2 | 9.5 | 26.9 | 4.8 |
| 17.2 | 27.6 | | |

### 1.2p-Toluenesulfonate Crystal Form I

At room temperature, about 30 mg of free base crystal form I was added to acetone (1.0 ml). After *p*-toluenesulfonic acid solution was added, the suspension turned into an almost yellow clear liquid, and became gelatinous after 5 minutes. After 1 hour, the solid precipitated, the mixture was stirred for another 2 days, and subjected to solid-liquid separation to obtain *p*-toluenesulfonate crystal form I.

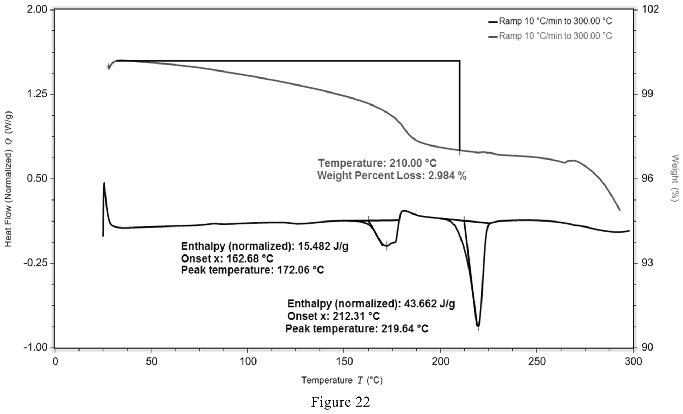

*p*-Toluenesulfonate crystal form I is an agglomerated fine crystal with high crystallinity. 1H-NMR shows 3.0% residual acetone with molar ratio of acid to base being 1:1. The DSC and TGA results are shown in Figure 22. The TGA result shows a weight loss of 3.0% before 210 °C, due to the removal of acetone. The DSC result shows a broad endothermic peak at 163 °C and another endothermic peak at 212 °C. Therefore, *p*-toluenesulfonate crystal form I may be a solvate.

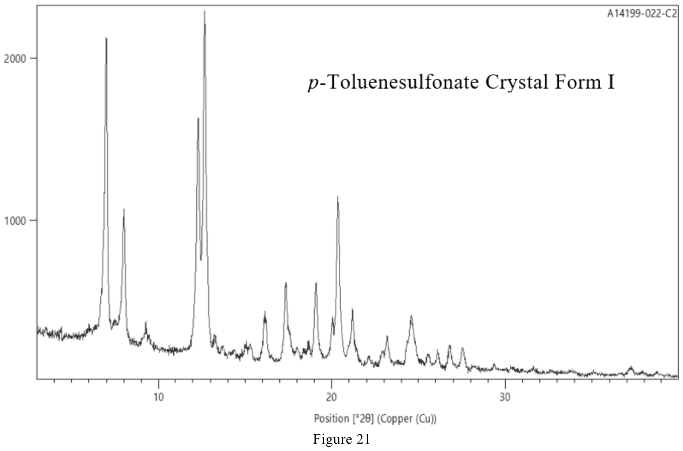

The powder X-ray diffraction pattern is shown in Figure 21, and the data is shown in Table 17:

**Table 17**

| 2θ (°) | Relative intensity (%) | 2θ (°) | Relative intensity (%) |
|---|---|---|---|
| 7.0 | 84.0 | 18.7 | 4.8 |
| 7.5 | 4.1 | 19.1 | 22.0 |
| 8.0 | 36.8 | 20.0 | 10.8 |
| 9.3 | 7.6 | 20.3 | 45.9 |
| 9.5 | 3.8 | 21.2 | 15.0 |
| 12.3 | 67.0 | 22.9 | 4.1 |
| 12.7 | 100.0 | 23.2 | 9.0 |
| 15.1 | 4.6 | 24.4 | 6.7 |
| 15.3 | 4.5 | 24.6 | 14.3 |
| 16.1 | 14.3 | 26.8 | 6.9 |
| 17.4 | 22.4 | 27.5 | 6.1 |

### 1.3 Methanesulfonate Crystal Form I

At room temperature, about 30 mg of free base crystal form I was added to acetone (1.0 ml). After methanesulfonic acid solution was added, the suspension turned into a yellow gel-like substance. After 1 hour, the solid precipitated, the mixture was stirred for 2 days and subjected to solid-liquid separation to obtain methanesulfonate crystal form I.

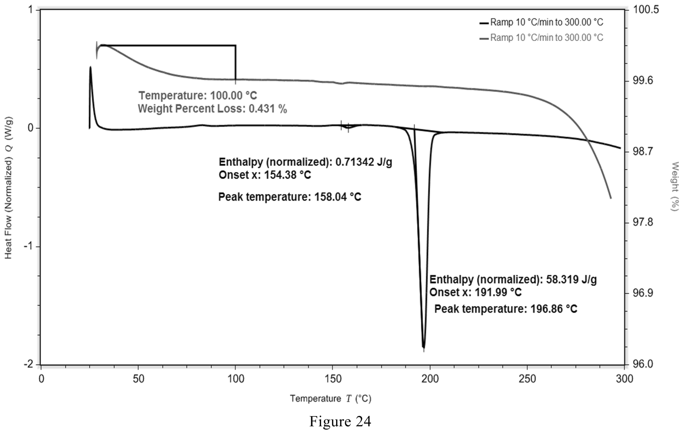

Methanesulfonate crystal form I is an agglomerated fine crystal with moderate crystallinity. 1H-NMR shows no significant residual organic solvents, with molar ratio of acid to base being 1:1. The DSC and TGA results are shown in Figure 24. The TGA result shows a 0.4% weight loss before 100 °C, attributed to the removal of water. The DSC result show a small endothermic peak at 154 °C and another endothermic peak at 192 °C, caused by sample melting. Therefore, the methanesulfonate crystal form I may be an anhydrous crystal form.

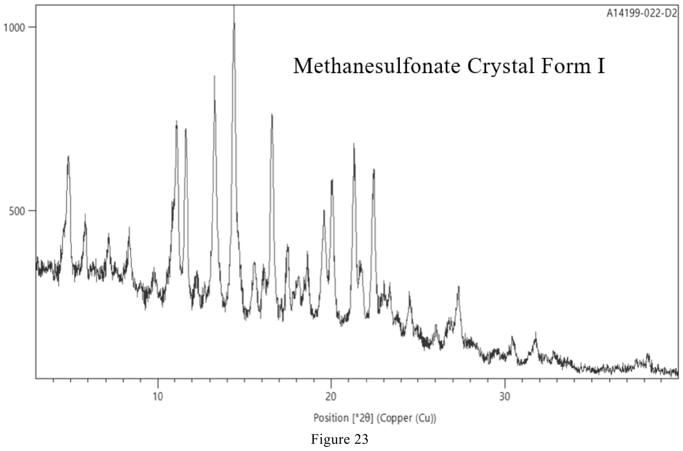

The powder X-ray diffraction pattern is shown in Figure 23, and the data is shown in Table 18:

**Table 18**

| 2θ (°) | Relative intensity (%) | 2θ (°) | Relative intensity (%) |
|---|---|---|---|
| 3.9 | 3.7 | 18.1 | 14.1 |
| 4.9 | 39.3 | 18.6 | 20.3 |
| 5.8 | 20.1 | 19.6 | 34.9 |
| 7.1 | 12.4 | 20.0 | 46.1 |
| 8.4 | 18.7 | 21.3 | 57.4 |
| 9.9 | 9.0 | 21.7 | 18.9 |
| 10.8 | 29.9 | 22.4 | 47.0 |
| 11.1 | 58.7 | 23.0 | 15.2 |
| 11.6 | 54.8 | 23.4 | 11.6 |
| 12.3 | 9.4 | 24.5 | 12.4 |
| 13.3 | 77.3 | 26.0 | 8.2 |
| 14.4 | 100.0 | 26.9 | 6.4 |
| 15.6 | 16.3 | 27.3 | 20.3 |
| 16.1 | 14.8 | 30.4 | 7.1 |
| 16.6 | 66.9 | 31.8 | 10.1 |
| 17.4 | 21.7 | | |

### 1.4 Benzenesulfonate Crystal form I

At room temperature, about 30 mg of free base crystal form I was added to acetone (1.0 ml). After benzenesulfonic acid solution was added, the suspension turned into an almost yellow clear liquid. After 30 seconds, a large amount of solid precipitated, and acetone (0.5 mL) was added. The mixture was stirred for 2 days and subjected to solid-liquid separation to obtain benzenesulfonate crystal form I.

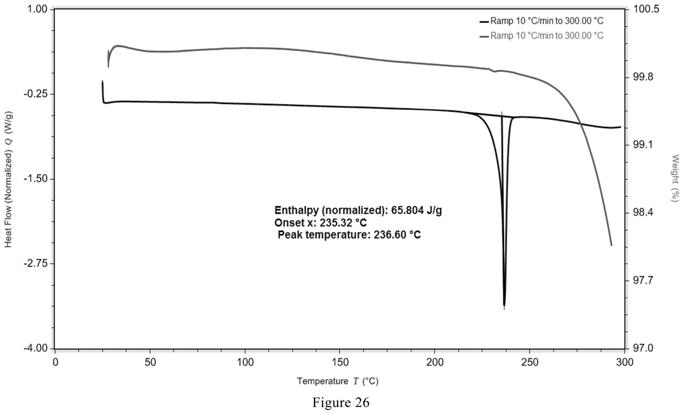

Benzenesulfonate crystal form I is an agglomerated fine crystal with high crystallinity. 1H-NMR shows 0.19% residual acetone, with molar ratio of acid to base being 1:1. The DSC and TGA results are shown in Figure 26. The TGA result shows no significant weight loss before 200 °C. The DSC result shows an endothermic peak at 235 °C, indicating the melting of the compound. Therefore, the benzenesulfonate crystal form I is an anhydrous crystal form.

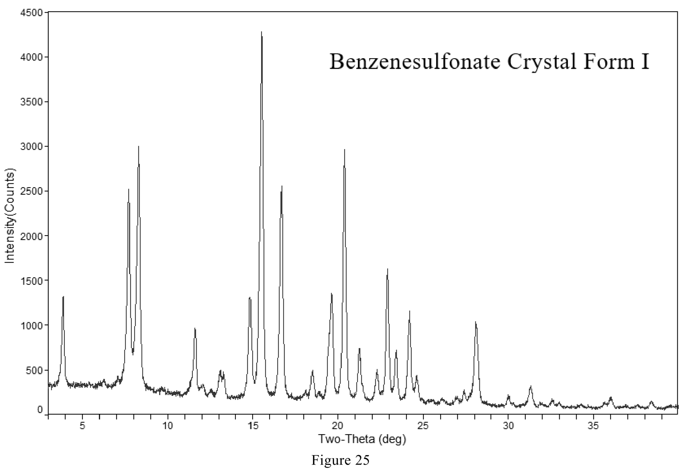

The powder X-ray diffraction pattern is shown in Figure 25, and the data is shown in Table 19:

**Table 19**

| 2θ (°) | Relative intensity (%) | 2θ (°) | Relative intensity (%) |
|---|---|---|---|
| 3.9 | 24.8 | 19.6 | 27.9 |
| 7.7 | 54.6 | 20.4 | 67.6 |
| 8.3 | 65.8 | 21.3 | 13.8 |
| 11.6 | 18.4 | 22.3 | 7.4 |
| 12.1 | 3.0 | 22.9 | 36.1 |
| 13.1 | 7.1 | 23.4 | 13.2 |
| 13.3 | 6.8 | 24.2 | 24.6 |
| 14.8 | 26.6 | 24.6 | 7.2 |
| 15.5 | 100.0 | 27.4 | 2.9 |
| 16.7 | 58.1 | 28.1 | 22.5 |
| 18.5 | 7.5 | 30.0 | 3.0 |
| | | 31.3 | 5.7 |

### 1.5 Oxalate Crystal Form I and Oxalate Crystal Form II

At room temperature, about 30 mg of free base crystal form I was added to methanol (1.0 ml). After oxalic acid solution was added, the suspension was stirred for another 2 days and subjected to solid-liquid separation to obtain oxalate crystal form I.

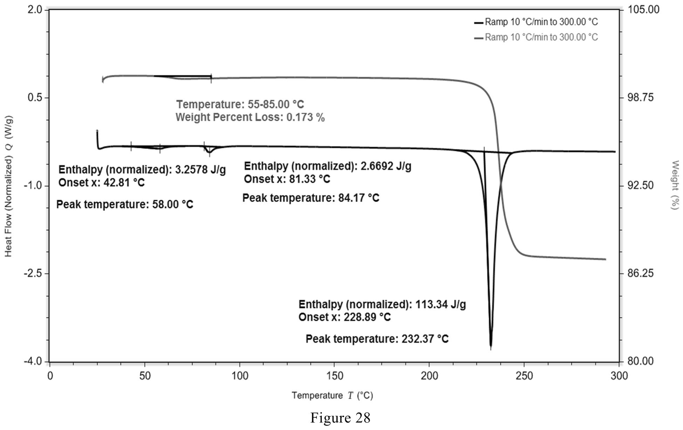

Oxalate crystal form I is a small particle crystal with high crystallinity. 1H-NMR shows no significant residual methanol. The DSC and TGA results are shown in Figure 28. The TGA result shows a weight loss of 0.2% between 55-85 °C, attributed to the removal of water. DSC shows two small endothermic peaks at 43 °C and 81 °C, and another endothermic peak at 229 °C, attributed to the dehydration and melting of water. Therefore, oxalate crystal form I may be an anhydrous crystal form.

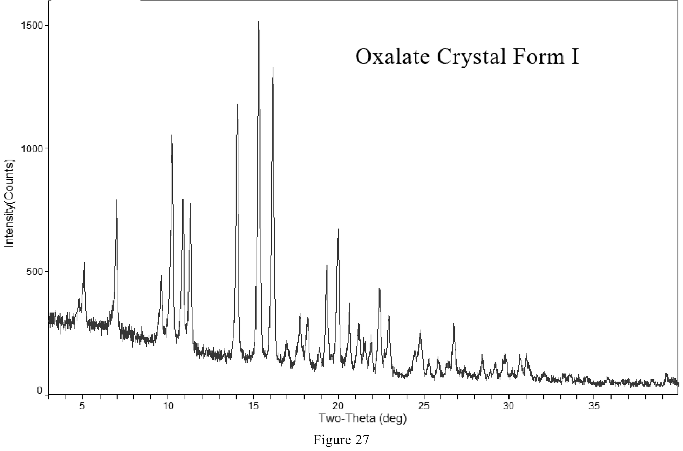

The powder X-ray diffraction pattern of oxalate crystal form I is shown in Figure 27, and the data is shown in Table 20:

**Table 20**

| 2θ (°) | Relative intensity (%) | 2θ (°) | Relative intensity (%) |
|---|---|---|---|
| 4.8 | 7.8 | 21.5 | 9.2 |
| 5.1 | 18.5 | 21.9 | 9.9 |
| 7.0 | 39.2 | 22.4 | 24.0 |
| 9.6 | 18.7 | 22.9 | 16.9 |
| 10.2 | 60.4 | 24.5 | 7.4 |
| 10.9 | 42.5 | 24.8 | 13.8 |
| 11.3 | 42.2 | 25.3 | 5.0 |
| 14.1 | 76.0 | 25.9 | 4.9 |
| 15.3 | 100.0 | 26.4 | 4.5 |
| 16.2 | 86.6 | 26.8 | 15.6 |
| 17.0 | 5.8 | 28.4 | 7.0 |
| 17.8 | 15.1 | 29.2 | 4.4 |
| 18.2 | 14.4 | 29.8 | 6.5 |
| 18.9 | 4.5 | 30.2 | 3.0 |
| 19.3 | 29.9 | 30.6 | 6.8 |
| 20.0 | 40.6 | 31.1 | 7.4 |
| 20.6 | 18.7 | 39.3 | 3.4 |
| 21.2 | 13.1 | | |

At room temperature, about 30 mg of free base crystal form I was added to acetone (1.0 ml). After oxalic acid solution (1.1 eq., 0.56 mol/L, oxalic acid dissolved in 0.1 mL corresponding solvent) was added, the suspension turned into a yellow gel-like substance. After 2 minutes, a large amount of solid precipitated, and acetone (0.5 mL) was added. The mixture was stirred for another 2 days and subjected to solid-liquid separation to obtain oxalate crystal form II.

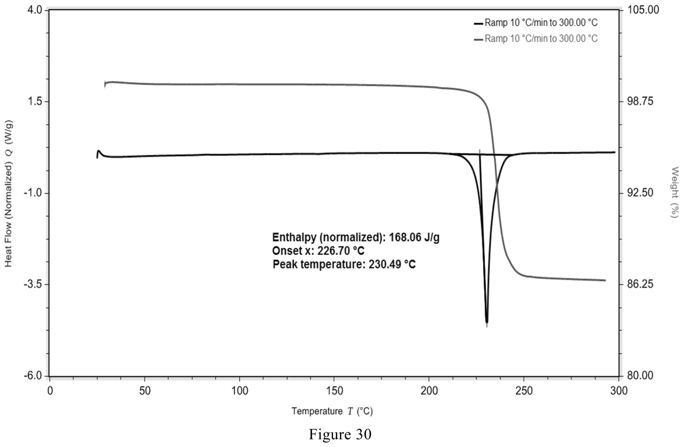

Oxalate crystal form II is an agglomerated fine crystal with high crystallinity. 1H-NMR shows 0.07% residual acetone. The DSC and TGA results are shown in Figure 30. TGA shows no significant weight loss before 200 °C. DSC shows an endothermic peak at 227 °C, caused by sample melting. Therefore, oxalate crystal form II is an anhydrous crystal form.

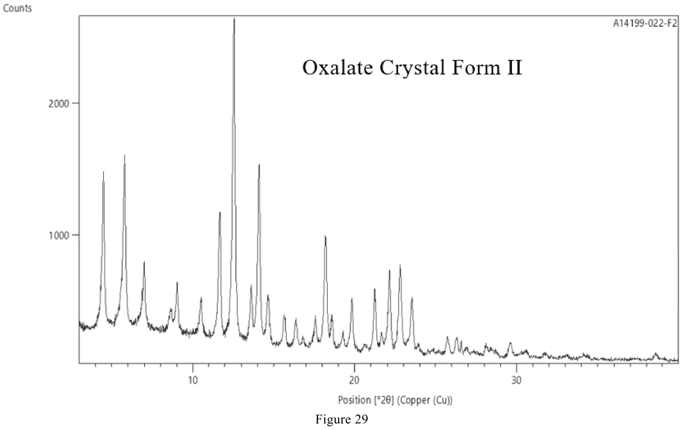

The powder X-ray diffraction pattern of oxalate crystal form II is shown in Figure 29, and the data is shown in Table 21:

**Table 21**

| 2θ (°) | Relative intensity (%) | 2θ (°) | Relative intensity (%) |
|---|---|---|---|
| 4.5 | 48.6 | 18.2 | 35.0 |
| 5.8 | 52.8 | 18.6 | 9.9 |
| 7.0 | 20.0 | 19.3 | 19.3 |
| 8.6 | 7.7 | 19.8 | 16.0 |
| 9.0 | 17.1 | 21.2 | 18.2 |
| 10.5 | 12.0 | 21.7 | 5.1 |
| 11.7 | 39.3 | 22.1 | 24.9 |
| 12.6 | 100.0 | 22.8 | 26.2 |
| 13.6 | 16.4 | 23.5 | 16.6 |
| 14.1 | 55.6 | 25.7 | 5.5 |
| 14.6 | 13.6 | 26.3 | 5.4 |
| 15.7 | 9.5 | 26.6 | 4.1 |
| 16.4 | 8.6 | 28.1 | 4.0 |
| 16.8 | 3.4 | 29.7 | 4.3 |
| 17.6 | 8.6 | | |

### 1.6 Maleate Crystal Form I

At room temperature, about 30 mg of free base crystal form I was added to 2-methyltetrahydrofuran (1.0 ml). After maleic acid solution (1.1 eq., 0.56 mol/L, maleic acid dissolved in 0.1 mL corresponding solvent) was added, the suspension was stirred for another 2 days, and subjected to solid-liquid separation to obtain maleate crystal form I.

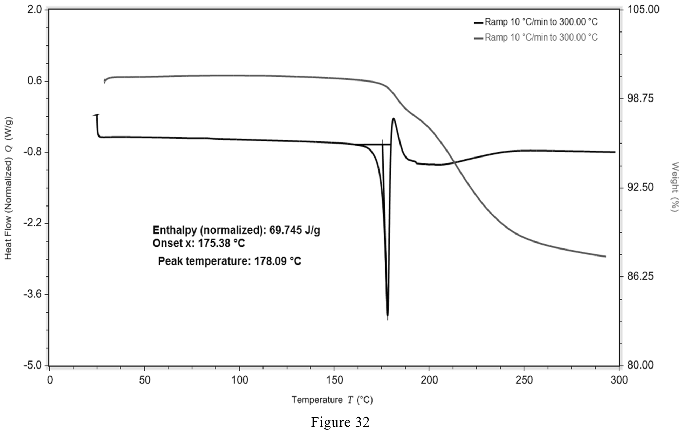

Maleate crystal form I is a small particle crystal with high crystallinity. 1H-NMR shows that the sample contains 0.41% residual 2-Me-THF, with molar ratio of acid to base being 1:1. The DSC and TGA results are shown in Figure 32. The TGA result shows no significant weight loss before 150 °C. DSC shows an endothermic peak at 175 °C, which is the melting point of the compound. Therefore, the maleate crystal form I is an anhydrous crystal form.

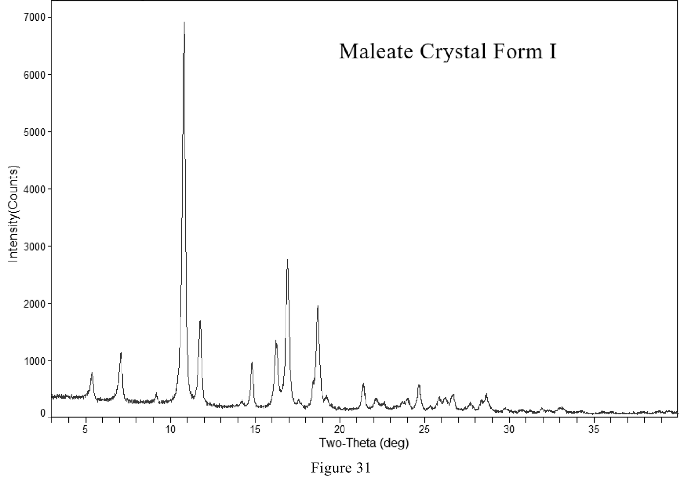

The powder X-ray diffraction pattern of maleate crystal form I is shown in Figure 31, and the data is shown in Table 22:

**Table 22**

| 2θ (°) | Relative intensity (%) | 2θ (°) | Relative intensity (%) |
|---|---|---|---|
| 5.4 | 6.9 | 19.2 | 3.1 |
| 7.1 | 12.6 | 21.4 | 6.6 |
| 9.2 | 2.6 | 22.1 | 2.7 |
| 10.8 | 100.0 | 24.7 | 6.5 |
| 11.8 | 20.9 | 25.9 | 3.3 |
| 14.8 | 11.8 | 26.3 | 3.2 |
| 16.2 | 16.9 | 26.7 | 3.8 |
| 16.9 | 38.6 | 28.7 | 4.6 |
| 18.7 | 27.0 | | |

### 1.7 Phosphate Crystal Form I

At room temperature, about 30 mg of free base crystal form I was added to the selected solvent. A phosphoric acid solution (1.1 eq., 0.56 mol/L, phosphoric acid dissolved in 0.1 mL corresponding solvent) was added to this suspension. The experimental process and results are shown in Table 23.

**Table 23 Preparation of Phosphate**

| Solvent (Vol.) | Acid (1.1 eq.) | Experimental process | Result |
|---|---|---|---|
| Acetone (1.0 mL) | H₃PO₄ solution | After phosphoric acid solution was added, the suspension turned yellow. After 1 hour, acetone (1.0 mL) was added and the mixture was stirred for 2 days | Phosphate crystal form I |
| 2-Me-THF (1.0 mL) | | After phosphoric acid solution was added, the suspension was stirred for 2 days | |

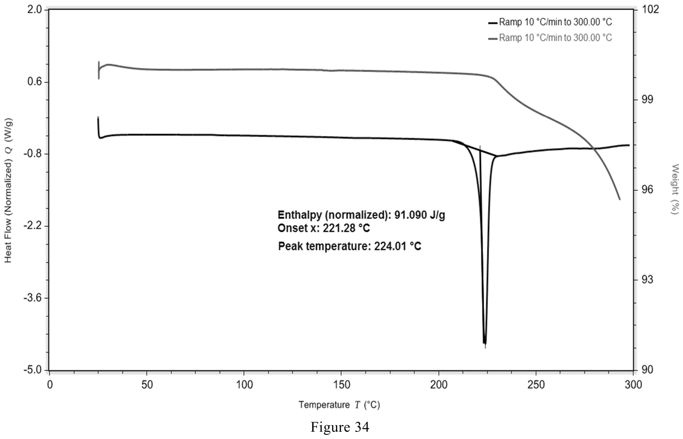

Phosphate crystal form I is a microcrystal with high crystallinity. 1H-NMR shows approximately 0.06% residual acetone. The DSC and TGA results are shown in Figure 34. The TGA result shows no significant weight loss before 200 °C. DSC shows an endothermic peak at 221 °C, which is attributed to the melting point of the compound. Therefore, phosphate crystal form I may be an anhydrous crystal form.

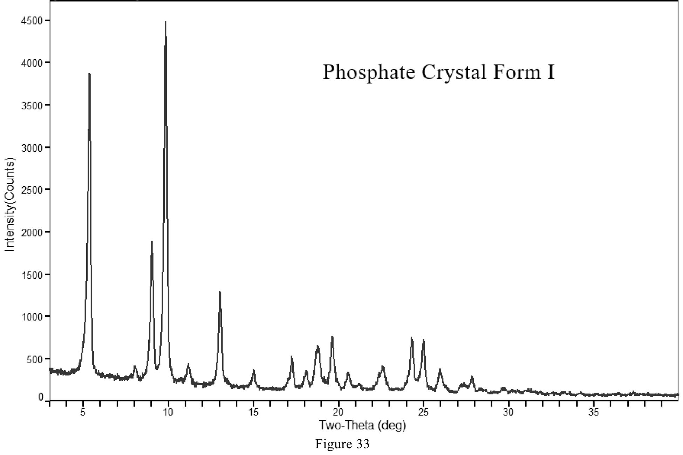

The powder X-ray diffraction pattern of phosphate crystal form I is shown in Figure 33, and the data is shown in Table 24:

**Table 24**

| 2θ (°) | Relative intensity (%) | 2θ (°) | Relative intensity (%) |
|---|---|---|---|
| 5.4 | 85.3 | 18.8 | 10.6 |
| 8.1 | 3.5 | 19.6 | 13.4 |
| 9.0 | 36.9 | 20.6 | 4.8 |
| 9.8 | 100.0 | 22.4 | 5.1 |
| 11.2 | 5.5 | 22.6 | 6.4 |
| 13.0 | 26.9 | 24.3 | 14.6 |
| 15.0 | 5.2 | 25.0 | 13.5 |
| 17.2 | 8.9 | 26.0 | 5.9 |
| 18.1 | 4.3 | 27.8 | 4.9 |

### 1.8 Tartrate Crystal Form I

At room temperature, about 30 mg of free base crystal form I was added to the selected solvent. A tartaric acid solid (1.1 eq.) was added to this suspension. The experimental process and result are shown in Table 25.

**Table 25 Preparation of Tartrate**

| Solvent (Vol.) | Acid (1.1 eq.) | Experimental process | Result |
|---|---|---|---|
| Acetone (1.0 mL) | TA | Tartaric acid was added, and after 1 hour, the mixture became gelatinous. Acetone (0.5 mL) was added, and the mixture was stirred for 2 days. | Tartrate crystal form I |
| 2-Me-THF (1.0 mL) | | Tartaric acid was added, and after 1 hour, the mixture became gelatinous. 2-Me-THF (0.5 mL) was added, and the mixture was stirred for 2 days. | |

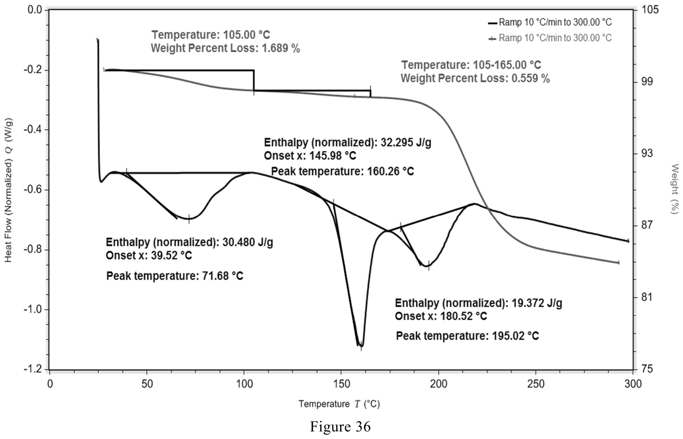

Tartrate crystal form I is an agglomerated fine crystal with moderate crystallinity. 1H-NMR shows approximately 0.08% residual acetone, with molar ratio of acid to base being 1:1. The DSC and TGA results are shown in Figure 36. The TGA result shows a 1.7% weight loss before 105 °C and a 0.6% weight loss between 105-165 °C. DSC shows a broad endothermic peak at 40 °C, and two other endothermic peaks at 146 °C and 181 °C, attributed to dehydration. Therefore, tartrate crystal form I may be a hydrate.

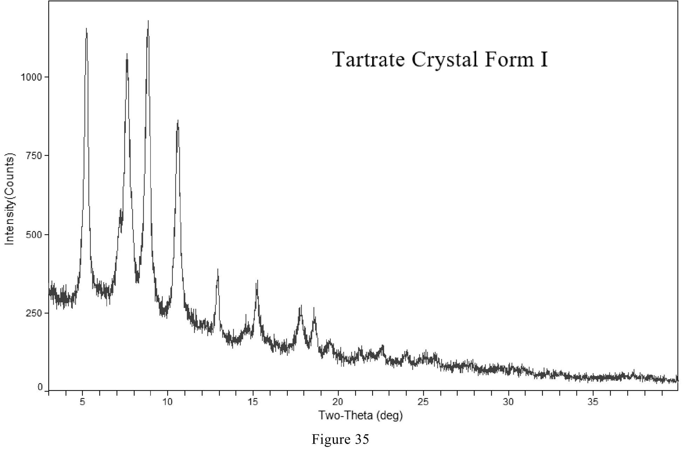

The powder X-ray diffraction pattern of tartrate crystal form I is shown in Figure 35, and the data is shown in Table 26:

**Table 26**

| 2θ (°) | Relative intensity (%) | 2θ (°) | Relative intensity (%) |
|---|---|---|---|
| 5.2 | 99.1 | 15.3 | 22.9 |
| 7.1 | 25.1 | 17.9 | 16.2 |
| 7.6 | 85.8 | 18.6 | 11.5 |
| 8.8 | 100.0 | 19.5 | 6.4 |
| 10.6 | 72.9 | 21.4 | 5.0 |
| 12.9 | 23.9 | 22.6 | 5.8 |
| | | 24.0 | 5.1 |

### 1.9 Fumarate Crystal Form I

At room temperature, about 30 mg of free base crystal form I was added to 2-Me-THF, and solid fumaric acid (1.1 eq.) was added to this suspension. The experimental process and result are shown in Table 27.

**Table 27 Preparation of Fumarate**

| Solvent (Vol.) | Acid (1.1 eq.) | Experimental process | Result |
|---|---|---|---|
| 2-Me-THF (1.0 mL) | FU | After fumaric acid was added, the suspension was stirred for 2 days. | Fumarate crystal form I |

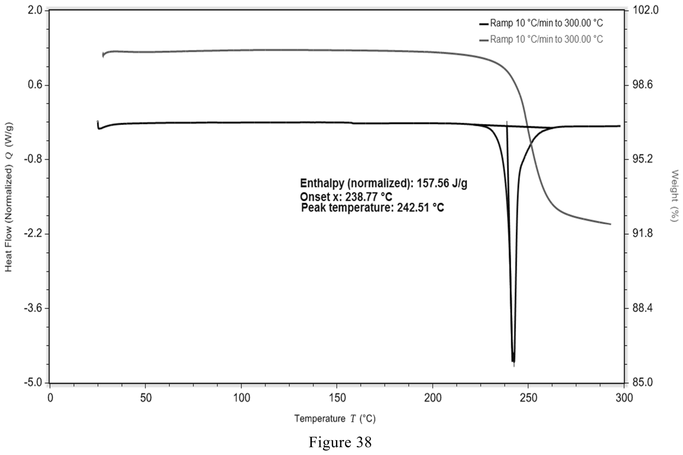

Fumarate crystal form I has agglomerated fine crystals with high crystallinity. 1H-NMR shows that the molar ratio of acid to base is 1:2. The DSC and TGA results are shown in Figure 38. The TGA result shows no significant weight loss before 200 °C. DSC shows an endothermic peak at 239 °C, which is attributed to the melting point of the compound. Therefore, fumarate crystal form I is an anhydrous crystal form.

The powder X-ray diffraction pattern of fumarate crystal form I is shown in Figure 37, and the data is shown in Table 28:

**Table 28**

| 2θ (°) | Relative intensity (%) | 2θ (°) | Relative intensity (%) |
|---|---|---|---|
| 6.0 | 100.0 | 16.5 | 4.1 |
| 8.2 | 2.8 | 17.5 | 19.8 |
| 8.7 | 10.5 | 18.0 | 14.2 |
| 9.9 | 3.0 | 19.2 | 21.5 |
| 10.6 | 15.2 | 21.2 | 4.0 |
| 11.0 | 9.4 | 22.2 | 3.0 |
| 11.9 | 6.8 | 23.9 | 4.7 |
| 12.1 | 9.4 | 25.5 | 3.1 |
| 12.5 | 6.4 | 26.7 | 8.1 |
| 14.6 | 12.4 | 27.1 | 11.3 |
| 15.5 | 12.3 | 28.8 | 4.7 |
| 15.9 | 6.8 | | |

### 1.10 Citrate Crystal Form I

At room temperature, about 30 mg of free base crystal form I was added to acetone (1.0 ml). Solid citric acid (1.1 eq.) was added to this suspension and the mixture was stirred for 2 days, and subjected to solid-liquid separation to obtain citrate crystal form I.

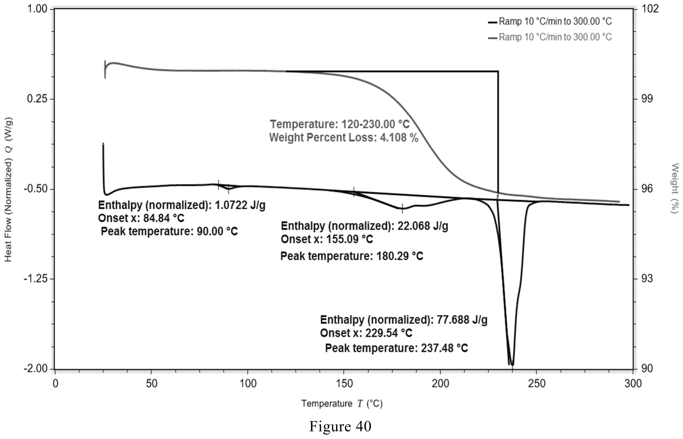

Citrate crystal form I has agglomerated fine crystals with moderate crystallinity. 1H-NMR shows approximately 0.08% residual acetone, with molar ratio of acid to base being 1:2. The DSC and TGA results are shown in Figure 40. The TGA result shows a weight loss of 4.1% between 120-230 °C, indicating the removal of water. DSC shows two broad endothermic peaks at 85 °C and 155 °C, and another endothermic peak at 230 °C, attributed to dehydration and melting point. Therefore, citrate crystal form I may be a hydrate.

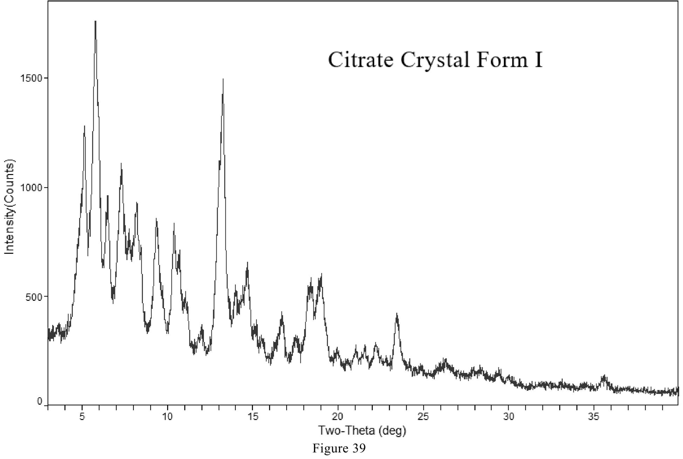

The powder X-ray diffraction pattern of citrate crystal form I is shown in Figure 39, and the data is shown in Table 29:

**Table 29**

| 2θ (°) | Relative intensity (%) | 2θ (°) | Relative intensity (%) |
|---|---|---|---|
| 3.7 | 5.6 | 15.2 | 7.5 |
| 5.1 | 81.5 | 15.5 | 5.1 |
| 5.8 | 94.9 | 16.7 | 18.3 |
| 6.5 | 32.5 | 17.5 | 7.2 |
| 7.3 | 44.5 | 18.4 | 29.1 |
| 7.8 | 32.3 | 19.0 | 31.1 |
| 8.2 | 50.8 | 20.0 | 5.2 |
| 9.4 | 44.1 | 21.1 | 8.4 |
| 10.4 | 44.6 | 21.6 | 7.2 |
| 10.7 | 35.8 | 22.2 | 8.7 |
| 11.1 | 15.4 | 23.4 | 20.8 |
| 12.0 | 10.5 | 26.3 | 6.2 |
| 13.2 | 100.0 | 28.0 | 4.0 |
| 14.0 | 14.3 | 29.5 | 4.5 |
| 14.7 | 28.6 | 35.6 | 5.6 |

### 1.11 Malate Crystal Form I

At room temperature, approximately 30 mg of free base crystal form I was added to the selected solvent. Malic acid solid (1.1 eq.) was added to this suspension. The experimental process and result are shown in Table 30.

**Table 30 Preparation of Malate**

| Solvent (Vol.) | Acid (1.1 eq.) | Experimental process | Result |
|---|---|---|---|
| 2-Me-THF (1.0 mL) | Malic acid | After malic acid was added, the mixture was stirred for 2 days | Malate crystal form I |

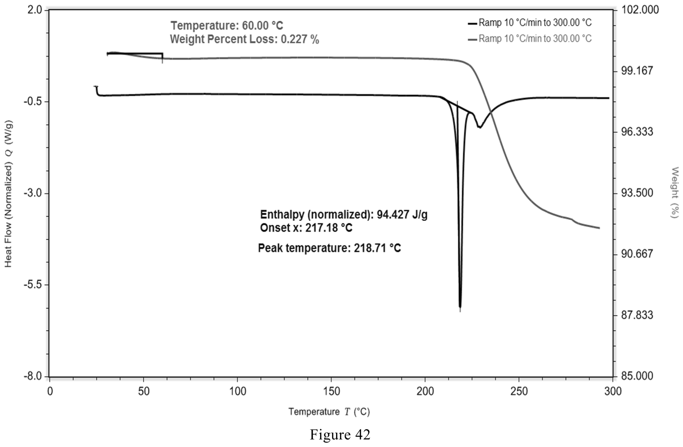

Malate crystal form I has agglomerated fine crystals with high crystallinity. 1H-NMR shows 0.06% residual acetone, with molar ratio of acid to base being 1:2. The DSC and TGA results are shown in Figure 42. The TGA result shows a 0.2% weight loss before 60 °C, attributed to the removal of water and acetone. DSC shows an endothermic peak at 217 °C, which is attributed to the melting point. Therefore, malate crystal form I is an anhydrous crystal form.

The powder X-ray diffraction pattern of malate crystal form I is shown in Figure 41, and the data is shown in Table 31:

**Table 31**

| 2θ (°) | Relative intensity (%) | 2θ (°) | Relative intensity (%) |
|---|---|---|---|
| 6.0 | 100.0 | 17.1 | 2.0 |
| 9.3 | 11.6 | 17.3 | 11.3 |
| 10.2 | 8.1 | 17.6 | 17.5 |
| 10.7 | 33.6 | 18.3 | 28.0 |
| 11.2 | 10.8 | 19.0 | 31.1 |
| 11.8 | 18.7 | 23.5 | 11.9 |
| 12.1 | 2.4 | 24.8 | 8.2 |
| 13.2 | 6.1 | 25.2 | 4.3 |
| 15.3 | 25.0 | 25.7 | 23.2 |
| 16.2 | 6.2 | 27.6 | 8.8 |
| 16.8 | 2.7 | 28.0 | 15.8 |
| | | 29.7 | 2.4 |

### 2. Analysis Method

### 2.1 Polarizing Microscope (PLM) Analysis

PLM analysis was performed using ECLIPSE LV100POL (Nikon, JPN) polarizing microscope. A small amount of sample was spread flat on a glass slide, cedar oil was dropped to disperse the sample, and then a cover glass was covered. Subsequently, the sample was placed under a microscope and observed with a 4-20 x objective lens.

### 2.2 X-ray Powder Diffraction (XRPD)

X-ray diffractometer was used to detect solid samples. The sample was spread flat on a zero background monocrystalline silicon sample disk, gently pressed and flatten, and analyzed according to the parameters in Table 32.

**Table 32 XRPD Test Parameters**

| | |
|---|---|
| Instrument | PANalytical, Empyrean |
| Light source | Cu Kα (λ = 1.5418 Å) |
| Detector | PIX_{cel}^{1D} |
| Scanning angle | 3-40° (2θ) |
| Scanning step size | 0.013° (2θ) |
| Scanning speed | 20.4 s/step |
| Light tube voltage/current | 45 kV/40 mA |
| Divergence slit | 1/8° |
| Rotate | open |
| Sample Disk | Zero Background Sample Disk |

### 2.3 Thermogravimetric Analysis (TGA)

The TGA data of the sample was collected using a TA instrument. 1-5 mg of the sample was placed in an open aluminum sample tray that has been tared, the sample was heated and tested according to the instrument parameters in Table 33, and the data was analyzed and processed using TRIOS software.

**Table 33. TGA Test Parameters**

| | |
|---|---|
| Instrument | TA, Discovery TGA 55 |
| Sample tray | Perforated aluminum tray |
| Temperature range | RT - 300 °C |
| Heating rate | 10 °C/min |
| Blow gas | N₂ |
| Flow rate | Balance Room: 40 mL/min |
| | Sample Room: 60 mL/min |

### 2.4 Differential Scanning Calorimetry (DSC) Analysis

The DSC data of the sample was collected using a TA instrument. 1-3 mg of the sample was placed in a DSC sample tray that has been perforated, heating measurement was performed according to the parameters in Table 34, and the data was analyzed and processed using TRIOS software.

**Table 34. DSC Test Parameters**

| | |
|---|---|
| Instrument | TA, Discovery DSC 250 |
| Sample tray | Perforated aluminum tray |
| Temperature range | 25-300 °C |
| Heating rate | 10 °C/min |
| Blow gas | N₂ |
| Flow rate | 50 mL/min |

### 2.5 Dynamic Water Vapor Sorption (DVS)

The water sorption and desorption analysis of the sample was performed using a DVS instrument. A certain amount of sample was placed in a tared sample tray, the sample was automatically weighed and analyzed according to the parameters in Table 35.

**Table 35 DVS Analysis Parameters for Anhydrous Crystal Form**

| | | |
|---|---|---|
| Instrument | ProUmid GmbH & Co. KG | |
| Sample temperature | 25 °C | |
| Cycle time | 10 min | |
| The minimum time for each cycle | 50 min | |
| The maximum duration for each cycle | 120 min | |
| Weight limit | 100% | |
| Equilibrium condition | 0.01%/45 min | |
| Cycle # 1 | 0%-0% | 40 °C 3 h, 1 step |
| Cycle # 2 | 0%-90% | 25 °C, 9 steps |
| Cycle # 3 | 80%-0% | 25 °C, 8 steps |
| Sorption | 0, 10, 20, 30, 40, 50, 60, 70, 80, 90 | |
| Desorption | 80, 70, 60, 50, 40, 30, 20, 10, 0 | |

### 2.6 Nuclear Magnetic Resonance Mass Spectrometry (¹H-NMR)

¹H-NMR data was collected using a Bruker 400 MHz instrument. DMSO-d6 was used as the solvent for the sample, and the test parameters are shown in Table 36. And the data was analyzed and processed using MestReNova software.

**Table 36 ¹H-NMR Test Parameters**

| | |
|---|---|
| Instrument | Bruker |
| Resolution | 400 mHz |
| Scanning time | 4 |
| Temperature | 295 K |
| Relaxation delay | 1 s |

### 2.7 High Performance Liquid Chromatography (HPLC)

HPLC analysis was performed using Agilent HPLC 1260 series instruments. The HPLC method for solubility and stability tests is shown in Table 37.

**Table 37 HPLC Method for Solubility and Stability Tests**

| | | | |
|---|---|---|---|
| Instruments | Agilent 1260 series | | |
| Column | Agilent Poroshell CS C18 4.6*150mm, 2.7um | | |
| Column temperature | 40 °C | | |
| Mobile phase | A: 0.02% aqueous TFA solution | | |
| | B: 0.02%TFA solution in acetonitrile | | |
| Flow rate | 1.0 mL/min | | |
| Injection volume | 1 µL | | |
| Detector& Wavelength | DAD; 210 nm | | |
| Running time | 18.1 min | | |
| Post-run time | 4.0 min | | |
| Diluent | MeOH | | |
| Gradient | Time (min) | %A | %B |
| | 0.0 | 95 | 5 |
| | 15.0 | 5 | 95 |
| | 18.0 | 5 | 95 |
| | 18.1 | 95 | 5 |

### 3. Evaluation of the acid salt and free base crystal forms of the compound shown in formula (A)

In this salt crystal form screening study, 8 anhydrous crystal forms were obtained: methanesulfonate, benzenesulfonate, 2 oxalates, maleate, phosphate, fumarate, and malate.

Based on the solid-state characterization data of salt and the screening results of free state, fumarate crystal form I, malate crystal form I, and free base crystal form III were selected for solubility, stability, and drug metabolism studies.

### 3.1 Solubility Study

Solubility tests were conducted on fumarate crystal form I and malate crystal form I in biological media and water at 37 °C. All results are shown in Table 38.

The maximum solubility of fumarate crystal form I and malate crystal form I occurs in SGF medium at 0.5 hours (0.21 mg/mL and 0.20 mg/mL, respectively), and the solubility decreases with time. All crystal forms are almost insoluble in water (<LOQ).

**Table 38 Solubility Test Results**

| Sample | Medium | Solubility (µg/mL)² | | | pH (0.5h/2.0h/24 h) | XRPD (24 h) |
|---|---|---|---|---|---|---|
| | | 0.5 h | 2 h | 24 h | | |
| Fumarate crystal form I | FaSSIF (pH=6.49) | <LOQ¹ | 5.950 | 22.662 | 6.48/6.54/5.91 | Almost amorphous |
| | FeSSIF (pH=5.00) | 84.190 | 28.508 | 20.924 | 5.01/5.03/4.93 | Unchanged |
| | SGF (pH=1.19) | 210.195 | 203.981 | 78.099 | 1.17/1.22/1.27 | Unchanged |
| | Water (pH=5.45) | <LOQ | | | 5.22/5.84/3.50 | Unchanged |
| Malate crystal form I | FaSSIF (pH=6.49) | <LOQ | 2.650 | <LOQ | 6.45/6.30/5.61 | Changed |
| | FeSSIF (pH=5.00) | 58.771 | 19.818 | 16.658 | 4.95/4.96/4.90 | Almost amorphous |
| | SGF (pH=1.19) | 204.753 | 113.155 | 79.977 | 1.27/1.29/1.33 | Changed |
| | Water (pH=5.45) | <LOQ | | | 4.76/4.19/3.88 | Unchanged |

| | | | | | | |
|---|---|---|---|---|---|---|
| LOQ: ~1.0 µg/mL The solubility of salt was calculated using free base. | | | | | | |

### 3.2 Solid State Stability Study

The solid-state stability of fumarate crystal form I, malate crystal form I, and free base crystal form III was tested under 60 °C/closed and 40 °C/75% RH conditions for 7 days. The experimental results are shown in Table 39.

According to Table 39, the crystal stability of fumarate crystal form I, malate crystal form I, and free base crystal form III is good, and three crystal forms of the compounds remain unchanged under both testing conditions. The chemical purity of fumarate crystal form I slightly decreases under 40 °C/75% RH condition, but its stability is good under closed high temperature condition, humidity should be controlled during storage. The other two crystal forms have good chemical stability under two conditions.

**Table 39 Stability Assessment Results**

| Sample | Initial purity (Area%) | Purity (Area%) | | XRPD | |
|---|---|---|---|---|---|
| | | 40 °C/ 75%RH | 60 °C/ Closed | 40 °C/ 75%RH | 60 °C/ Closed |
| Free base crystal form III | 99.316 | 99.253 | 99.212 | Unchanged | Unchanged |
| Fumarate crystal form I | 98.062 | 97.914 | 98.119 | Unchanged | Unchanged |
| Malate crystal form I | 98.772 | 98.742 | 98.753 | Unchanged | Unchanged |

### 3.3 Evaluation of Amorphous Free Base

100 mg of the amorphous free base sample obtained in the present invention was weighed and a solvent was added to form a suspension. The mixture was stirred at room temperature for 2 days, and the results are as follows:

| Solvent | Experiment process | Result |
|---|---|---|
| Methanol | After methanol was added, the suspension was stirred at room temperature for another 2 days | Free base crystal form I |
| Acetone | After acetone was added, the suspension was stirred at room temperature for another 2 days | Free base crystal form III |

From the above results, it can be concluded that the amorphous sample of free base is unstable and easily transformed into other crystal forms in the solvent.

### 3.4 Drug Metabolism Research

*In vivo* drug metabolism experiments were conducted in rats and dogs on fumarate crystal form I, malate crystal form I, free base crystal form I, and free base crystal form III.

SD rats were administered with fumarate crystal form I, malate crystal form I, free base crystal form I, and free base crystal form III by gavage in a single dose. Blood samples were collected at different time points to determine the drug concentration in the rat plasma after administration of the test substance and relevant pharmacokinetic parameters were calculated.

Dogs were administered with fumarate crystal form I, free base crystal form I, and free base crystal form III by gavage in a single dose. Blood samples were collected at different time points to determine the drug concentration in the dog plasma after administration of the test substance and relevant pharmacokinetic parameters were calculated.

### 3.4.1 Preparation of Test Sample Solution

The preparation method was as follows:
The solvents for the code names fumarate crystal form I, malate crystal form I, free base crystal form I, and free base crystal form III were 0.5% MC+1% Tween 80. The specific preparation method was as follows: 10mg of the test sample was taken, 0.5% MC and 1% Tween 80 was added, the resulting mixture was vortex mixed to obtain a solution with a concentration of 1mg/mL. The solution was freshly prepared before use, mixed well through vortex, and set aside for later use.

### 3.4.2 Analysis of Test Sample Solution

The prepared test sample solution was analyzed by LC-MS/MS in the analysis department of our experimental institution.

### 3.4.3 Animal Reception and Adaptation

Healthy male SD rats or dogs were used for this study. All animals were fasted before administration and resumed feeding 4 hours after administration.

### 3.4.4 Sample Collection and Processing

The time points for blood collection were:
Oral administration: 0.25, 0.5, 1, 2, 4, 6, 8, 12 and 24 hours after administration. (Time points ≤ 2 hours: ± 2 min; other points: ± 15 min.)

About 0.1 mL of blood was collected via the jugular vein from each animal, using EDTA or heparin sodium as anticoagulants. After collected, the blood sample was placed on ice and centrifuged to separate the plasma (centrifugation conditions: 10000 rpm, 5 minutes, and 4 °C).

The plasma samples were analyzed by LC-MS/MS by the analysis department of the experimental institution.

### 3.4.5 Pharmacokinetic Analysis

Based on the blood drug concentration data of the drug, the pharmacokinetic parameters AUC_{0→t}, AUC_{0→∞}, Cₘₐₓ, Tₘₐₓ, T_{1/2}, as well as their means and standard deviations, were calculated using the WinNonlin non-compartmental model pharmacokinetic calculation software.

### 3.4.6 Experimental Results

The main pharmacokinetic parameters in plasma after a single oral administration of fumarate crystal form I, malate crystal form I, free base crystal form I, and free base crystal form III are shown in Tables 40 and 41.

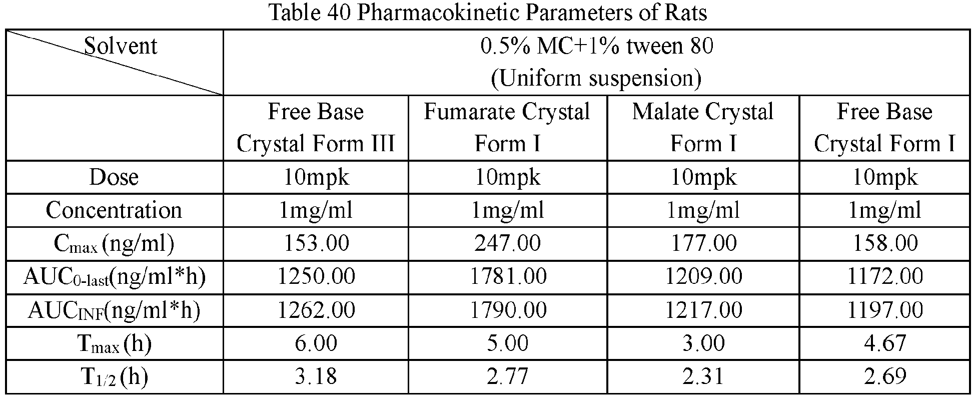

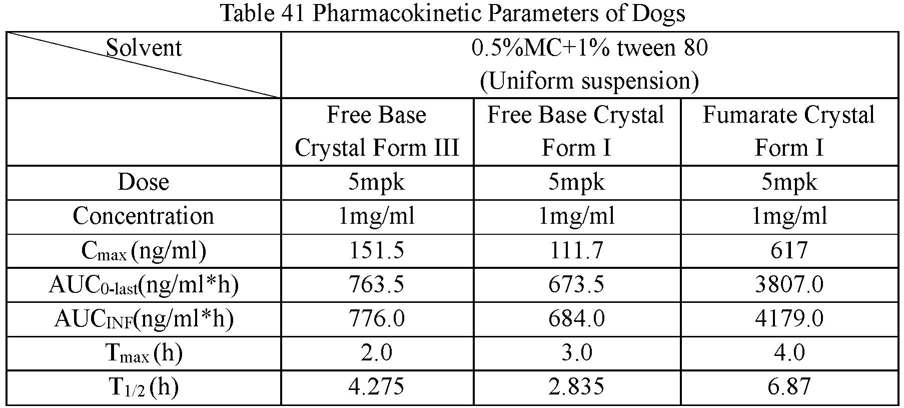

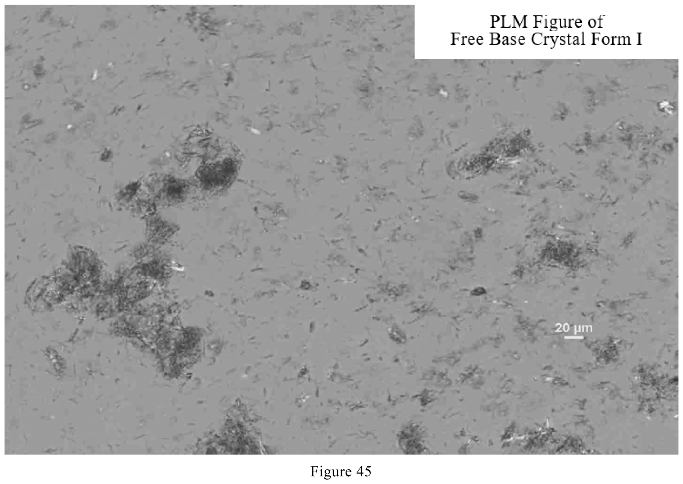

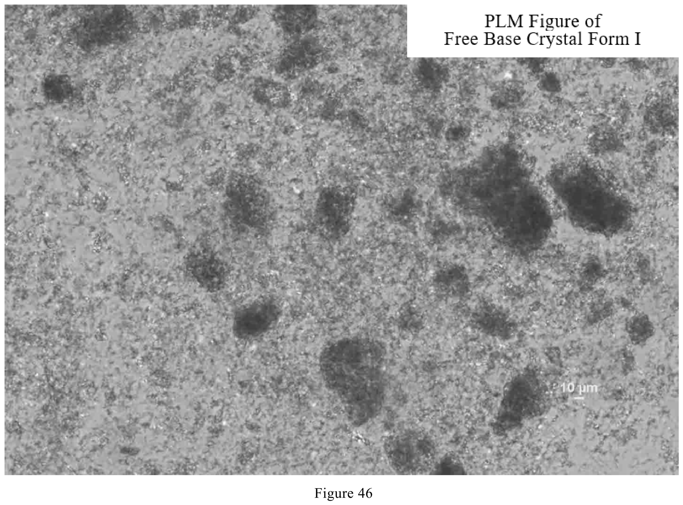

The above pharmacokinetic results show that the *in vivo* exposure levels of fumarate crystal form I, malate crystal form I, and free base crystal form III are higher than those of free base crystal form I in both rat and dog pharmacokinetic data. The PLM diagrams of two batches of free base crystal form I are shown in Figure 45 and Figure 46. From the figures, it can be seen that they are agglomerates with very small particle size, which are very difficult to be filtered in the actual production process and are not conducive to large-scale production.

In summary, fumarate crystal form I and malate crystal form I have excellent *in vivo* exposure levels in animals, good solid-state stability, and are suitable for large-scale production, making them suitable for further research and development.

All documents mentioned herein are incorporated by reference in this application as if each document is individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. A crystal form of a compound of formula A, wherein the crystal form is selected from the group consisting of free base crystal form III, fumarate crystal form I, and malate crystal form I, 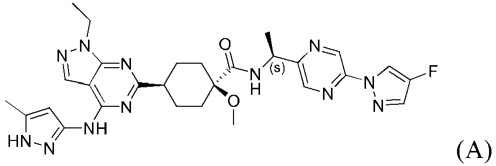 wherein,
the X-ray powder diffraction pattern of the free base crystal form III exhibits characteristic peaks at the following 2θ values: 5.1±0.2°, 5.8±0.2°, 7.3±0.2°, 8.2±0.2°, 9.3±0.2°, 10.4±0.2°, 10.7±0.2°, 13.0±0.2°, 13.3±0.2°, 14.7±0.2°, 16.7±0.2°, 18.4±0.2°, 19.0±0.2° and 23.4±0.2°;
the X-ray powder diffraction pattern of fumarate crystal form I exhibits characteristic peaks at the following 2θ values: 6.0±0.2°, 8.7±0.2°, 10.6±0.2°, 14.6±0.2°, 15.5±0.2°, 17.5±0.2°, 18.0±0.2°, 19.2±0.2° and 27.1±0.2°;
the X-ray powder diffraction pattern of malate crystal form I exhibits characteristic peaks at the following 2θ values: 6.0±0.2°, 9.3±0.2°, 10.7±0.2°, 11.2±0.2°, 11.8±0.2°, 15.3±0.2°, 17.3±0.2°, 17.6±0.2°, 18.3±0.2°, 19.0±0.2°, 23.5±0.2°, 25.7±0.2° and 28.0±0.2°.

2. The crystal form according to claim 1, wherein
the X-ray powder diffraction pattern of the free base crystal form III exhibits characteristic peaks at the following 2θ values: 5.1±0.2°, 5.8±0.2°, 7.3±0.2°, 7.8±0.2°, 8.2±0.2°, 9.3±0.2°, 10.4±0.2°, 10.7±0.2°, 11.0±0.2°, 13.0±0.2°, 13.3±0.2°, 14.0±0.2°, 14.7±0.2°, 15.2±0.2°, 16.7±0.2°, 17.5±0.2°, 18.4±0.2°, 19.0±0.2°, 21.1±0.2°, 22.2±0.2°, 23.4±0.2°and 29.5±0.2°;
the X-ray powder diffraction pattern of fumarate crystal form I exhibits characteristic peaks at the following 2θ values: 6.0±0.2°, 8.7±0.2°, 10.6±0.2°, 11.0±0.2°, 11.9±0.2°, 12.1±0.2°, 12.5±0.2°, 14.6±0.2°, 15.5±0.2°, 15.9±0.2°, 17.5±0.2°, 18.0±0.2°, 19.2±0.2°, 26.7±0.2°and 27.1±0.2°;
the X-ray powder diffraction pattern of malate crystal form I exhibits characteristic peaks at the following 2θ values: 6.0±0.2°, 9.3±0.2°, 10.2±0.2°, 10.7±0.2°, 11.2±0.2°, 11.8±0.2°, 13.2±0.2°, 15.3±0.2°, 16.2±0.2°, 17.3±0.2°, 17.6±0.2°, 18.3±0.2°, 19.0±0.2°, 23.5±0.2°, 24.8±0.2°, 25.7±0.2°, 27.6±0.2°and 28.0±0.2°.

3. The crystal form according to claim 1, wherein
the X-ray powder diffraction pattern of the free base crystal form III exhibits characteristic peaks at the following 2θ values: 5.1±0.2°, 5.8±0.2°, 7.3±0.2°, 7.8±0.2°, 8.2±0.2°, 9.3±0.2°, 10.4±0.2°, 10.7±0.2°, 11.0±0.2°, 13.0±0.2°, 13.3±0.2°, 14.0±0.2°, 14.7±0.2°, 15.2±0.2°, 16.7±0.2°, 17.5±0.2°, 18.4±0.2°, 19.0±0.2°, 21.1±0.2°, 21.6±0.2°, 22.2±0.2°, 23.4±0.2°, 24.9±0.2°, 25.9±0.2°, 26.3±0.2°, 27.9±0.2°, 28.4±0.2°, 29.5±0.2°, 30.0±0.2°and 35.6±0.2°;
the X-ray powder diffraction pattern of fumarate crystal form I exhibits characteristic peaks at the following 2θ values: 6.0±0.2°, 8.2±0.2°, 8.7±0.2°, 9.9±0.2°, 10.6±0.2°, 11.0±0.2°, 11.9±0.2°, 12.1±0.2°, 12.5±0.2°, 14.6±0.2°, 15.5±0.2°, 15.9±0.2°, 16.5±0.2°, 17.5±0.2°, 18.0±0.2°, 19.2±0.2°, 21.2±0.2°, 22.2±0.2°, 23.9±0.2°, 25.5±0.2°, 26.7±0.2°, 27.1±0.2°and 28.8±0.2°;
the X-ray powder diffraction pattern of malate crystal form I exhibits characteristic peaks at the following 2θ values: 6.0±0.2°, 9.3±0.2°, 10.2±0.2°, 10.7±0.2°, 11.2±0.2°, 11.8±0.2°, 12.1±0.2°, 13.2±0.2°, 15.3±0.2°, 16.2±0.2°, 16.8±0.2°, 17.1±0.2°, 17.3±0.2°, 17.6±0.2°, 18.3±0.2°, 19.0±0.2°, 23.5±0.2°, 24.8±0.2°, 25.2±0.2°, 25.7±0.2°, 27.6±0.2°, 28.0±0.2°and 29.7±0.2°.

4. The crystal form according to claim 1, wherein
the X-ray powder diffraction pattern of the free base crystal form III is substantially shown in Figure 5;
the X-ray powder diffraction pattern of the fumarate crystal form I is substantially shown in Figure 37;
the X-ray powder diffraction pattern of the malate crystal form I is substantially shown in Figure 41.

5. The crystal form according to claim 1, wherein the crystal form is selected from the group consisting of fumarate crystal form I and malate crystal form I.

6. The crystal form according to claim 1, wherein the crystal form is fumarate crystal form I.

7. A method for preparing free base crystal form III, wherein the method is selected from the group consisting of:
method 1: mixing a compound of formula A with a solvent m, dissolving and clarifying, and crystallizing; the solvent m is selected from one of butanone, acetone, and acetonitrile; and
method 2: mixing a compound of formula A with a solvent n, slurrying at high temperature, and crystallizing; the solvent n is selected from one of ethyl acetate, isopropyl acetate, and methyl *tert*-butyl ether.

8. A method for preparing fumarate crystal form I or malate crystal form I, wherein the method is selected from the group consisting of:
method 1: dissolving a compound of formula A in an organic solvent, stirring at room temperature, then adding fumaric acid or malic acid, continuing stirring, and performing solid-liquid separation to obtain the fumarate crystal form I or malate crystal form I; and
method 2: adding an organic solvent to the fumarate or malate of compound A, suspending and stirring, and performing solid-liquid separation to obtain the fumarate crystal form I or malate crystal form I.

9. A use of the crystal form according to claim 1 for the manufacture of a medicament, the medicament is used for a use selected from the group consisting of:
1) treating a disease or condition related to RET regulation;
2) treating a tumor; and
3) inhibiting cell proliferation.

10. The use according to claim 9, wherein the tumor is selected from the group consisting of thyroid cancer and non-small cell lung cancer.
